(19) Europäisches Patentamt
European Patent Office
Office européen des brevets

(11) **EP 4 563 548 A1**

(12) **EUROPEAN PATENT APPLICATION**
published in accordance with Art. 153(4) EPC

(43) Date of publication:
**04.06.2025 Bulletin 2025/23**

(21) Application number: **24779946.3**

(22) Date of filing: **22.03.2024**

(51) International Patent Classification (IPC):
**C04B 35/488** (2006.01)    **A61C 8/00** (2006.01)
**A61K 6/818** (2020.01)    **C01G 25/02** (2006.01)

(52) Cooperative Patent Classification (CPC):
**A61C 8/00; A61K 6/818; C01G 25/02; C04B 35/488**

(86) International application number:
**PCT/JP2024/011255**

(87) International publication number:
**WO 2024/203828 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023057562**

(71) Applicant: **Daiichi Kigenso Kagaku Kogyo Co., Ltd.**
**Osaka-shi, Osaka 541-0041 (JP)**

(72) Inventors:
• **KOMATSU, Yuki**
**Osaka-shi, Osaka 559-0025 (JP)**
• **TAKAI, Masayuki**
**Osaka-shi, Osaka 559-0025 (JP)**

(74) Representative: **Cabinet Beau de Loménie**
**158, rue de l'Université**
**75340 Paris Cedex 07 (FR)**

(54) **SILVER-CONTAINING ZIRCONIA SINTERED BODY, SILVER-CONTAINING STABILIZED ZIRCONIA POWDER, METHOD FOR PRODUCING SILVER-CONTAINING STABILIZED ZIRCONIA POWDER, AND METHOD FOR PRODUCING SILVER-CONTAINING ZIRCONIA SINTERED BODY**

(57) Provided is a silver-containing zirconia sintered body comprising silver particles, zirconia, and a stabilizing agent, wherein the silver particles have an average particle diameter of 0.5 μm or less, stabilized zirconia has an average crystal grain diameter of 0.35 μm or less, and the relative sintered density is 98% or more.

Fig.1

EP 4 563 548 A1

**Description**

TECHNICAL FIELD

The present invention relates to a silver-containing zirconia sintered body, a silver-containing stabilized zirconia powder, a method for producing a silver-containing stabilized zirconia powder, and a method for producing a silver-containing zirconia sintered body.

BACKGROUND ART

As a method for imparting antibacterial action to zirconia ceramics (zirconia sintered bodies), it is common to coat the ceramic surface with an antibacterial substance. As antibacterial substances, silver, which has a wide antibacterial spectrum, and titanium oxide, which functions as a photocatalyst, are used.

Silver is used in prostheses such as dental implants in order to suppress the formation of biofilms that lead to infection. Non-Patent Documents 1 and 2 disclose that antibacterial action is imparted to prostheses by coating the prostheses with silver nanoparticles.

Conventionally, ceramic knives to which antibacterial action is imparted by enamel coating the surface with titanium oxide have been sold.

PRIOR ART DOCUMENT

NON-PATENT DOCUMENTS

Non-Patent Document 1: Kaijin Xu, Ceram. Int., 2011, 37, p2109-2115. "Microorganism adhesion inhibited by silver doped Yttria-stabilized zirconia ceramics"

Non-Patent Document 2: Yamada, R., Mater. Sci. Eng. C, 2017, 78, p1054-1060. "Ag nanoparticle-coated zirconia for antibacterial prosthesis"

SUMMARY OF THE INVENTION

PROBLEMS TO BE SOLVED BY THE INVENTION

However, coatings as described above may peel off by wear, and the antibacterial action is hardly durable.

As a result of extensive investigation, the present inventors have come to the conclusion that it may be possible to suppress the decrease in antibacterial action due to wear if antibacterial substances can be incorporated into the system of zirconia sintered bodies.

Silver is said to have a broader antibacterial spectrum and stronger antibacterial action than copper and zinc, which also have antibacterial action. However, as a result of further investigation by the present inventors, it has become clear that the mechanical properties, sinterability, and resistance to hydrothermal degradation significantly decrease when silver and stabilized zirconia are mixed to prepare a silver-containing zirconia powder and the silver-containing zirconia powder is sintered to produce a zirconia sintered body.

The sinterability and resistance to hydrothermal degradation of copper and zinc do not decrease even when copper and zinc are mixed with stabilized zirconia and sintered.

The present invention has been devised in view of the above-described problems, and an object thereof is to provide a silver-containing zirconia sintered body that is excellent in mechanical properties, sinterability, and resistance to hydrothermal degradation. Another object of the present invention is to provide a silver-containing stabilized zirconia powder that can be converted into the silver-containing zirconia sintered body by being sintered. Another object of the present invention is to provide a method for producing the silver-containing stabilized zirconia powder. Still another object of the present invention is to provide a method for producing the silver-containing zirconia sintered body.

MEANS FOR SOLVING THE PROBLEMS

In order to solve the above problems, the present inventors have conducted extensive studies on a silver-containing zirconia sintered body. As a result, the present inventors have found out that it is possible to provide a silver-containing zirconia sintered body that is excellent in mechanical properties, sinterability, and resistance to hydrothermal degradation by adopting the following configuration, and thus completed the present invention.

In other words, the present invention provides the following.

[1] A silver-containing zirconia sintered body containing:

silver particles; and
stabilized zirconia containing zirconia and a stabilizer, in which
an average particle diameter of the silver particles is 0.5 $\mu$m or less,
an average crystal grain size of the stabilized zirconia is 0.35 $\mu$m or less, and
a relative sintered density is 98% or more.

According to the configuration of [1], the surface of the sintered body is not coated with silver particles but silver particles are contained in the silver-containing zirconia sintered body itself. Therefore, when the surface of the silver-containing zirconia sintered body is worn as well, silver particles are exposed from the inside, and the antibacterial action can be maintained.

Silver is excellent in ductility and malleability, so it is difficult to atomize silver by pulverization. However, in the present invention, the silver particles in the silver-containing zirconia sintered body can be atomized by adopting the production method described below. Specifically, the average particle diameter of silver particles can be set to 0.5 $\mu$m or less. When silver particles are fine particles, segregation of silver particles in the silver-containing zirconia sintered body can be suppressed. As a result, sintering at a low temperature is possible. In addition, the decrease in mechanical properties is suppressed. In other words, as the dispersibility of silver particles in the silver-containing zirconia sintered body is improved, densification is possible (it is possible to increase the relative sintered density), and the decrease in mechanical properties of the silver-containing zirconia sintered body is suppressed. Specifically, by setting the relative sintered density to 98% or more, the decrease in mechanical properties of the silver-containing zirconia sintered body is suppressed.

The relative sintered density can be improved by setting the sintering temperature to a high temperature (for example, more than 1500°C), but the crystal grain size of zirconia increases and the resistance to hydrothermal degradation decreases when the sintering temperature is set to a high temperature.

However, in the present invention, the average crystal grain size of stabilized zirconia is set to 0.35 $\mu$m or less by setting the sintering temperature to a relatively low temperature. As a result, the decrease in the resistance to hydrothermal degradation is suppressed.

As described above, according to the configuration of [1], by setting the average particle diameter of silver particles to 0.5 $\mu$m or less, the decrease in sinterability is suppressed, and densification is possible (relative sintered density is 98% or more), and the decrease in mechanical properties is suppressed. Since the average crystal grain size of stabilized zirconia is 0.35 $\mu$m or less, the decrease in the resistance to hydrothermal degradation is suppressed.

Furthermore, the present invention provides the following.

[2] The silver-containing zirconia sintered body according to [1], in which an average crystal grain size of the stabilized zirconia is 0.25 $\mu$m or less.

When the average crystal grain size of the stabilized zirconia is 0.25 $\mu$m or less, it can be said that the sintering temperature is lower.

Furthermore, the present invention provides the following.

[3] The silver-containing zirconia sintered body according to [1] or [2], in which silver particles are contained in a range of 0.025% by mass or more and 20% by mass or less in terms of $Ag_2O$ when a sum of zirconia and a stabilizer is taken as 100% by mass.

When the content of the silver particles is 0.025% by mass or more, the silver-containing zirconia sintered body is excellent in antibacterial properties. When the content of the silver particles is 20% by mass or less, the silver-containing zirconia sintered body is superior in mechanical properties and sinterability.

Furthermore, the present invention provides the following.

[4] The silver-containing zirconia sintered body according to any one of [1] to [3], which has a three-point bending strength of 600 MPa or more.

When the three-point bending strength is 600 MPa or more, it can be said that the strength of the silver-containing zirconia sintered body is higher.

Furthermore, the present invention provides the following.

[5] The silver-containing zirconia sintered body according to any one of [1] to [4], which has a toughness value of 5 MPa·m$^{0.5}$ or more as measured by an IF method.

When the toughness value is 5 MPa·m$^{0.5}$ or more, it can be said that the silver-containing zirconia sintered body has higher toughness.

Furthermore, the present invention provides the following.

[6] The silver-containing zirconia sintered body according to any one of [1] to [5], in which a monoclinic fraction after hydrothermal treatment at 134°C and 0.3 MPa for 15 hours is 10% or less.

When the monoclinic fraction after hydrothermal treatment at 134°C and 0.3 MPa for 15 hours is 10% or less, it can be said that the silver-containing zirconia sintered body is superior in resistance to hydrothermal degradation.

Furthermore, the present invention provides the following.

[7] The silver-containing zirconia sintered body according to any one of [1] to [6], in which the stabilizer is one or more selected from the group consisting of $Y_2O_3$, $CeO_2$, $Er_2O_3$, and CaO.

When the stabilizer is one or more selected from the group consisting of $Y_2O_3$, $CeO_2$, $Er_2O_3$, and CaO, the silver-containing zirconia sintered body is superior in low temperature sinterability and mechanical strength.

Furthermore, the present invention provides the following.

[8] The silver-containing zirconia sintered body according to any one of [1] to [7], in which a content of a stabilizer is 15 mol% or less when zirconia and the stabilizer are taken as the whole.

When the content of the stabilizer is 15 mol% or less, the silver-containing zirconia sintered body is superior in mechanical strength.

Furthermore, the present invention provides the following.

[9] A silver-containing stabilized zirconia powder containing:

stabilized zirconia containing zirconia and a stabilizer; and
silver and/or silver oxide having an average particle diameter of 0.5 $\mu$m or less.

Silver oxide particles are converted into silver particles when thermally decomposed. According to the silver-containing stabilized zirconia powder according to [9], since stabilized zirconia and silver and/or silver oxide having an average particle diameter of 0.5 $\mu$m or less are contained, a sintered body containing silver (silver particles) having an average particle diameter of 0.5 $\mu$m or less can be obtained when the silver-containing stabilized zirconia powder is sintered.

In other words, in any one of a case where the silver-containing stabilized zirconia powder contains only silver oxide having an average particle diameter of 0.5 $\mu$m or less, a case where the silver-containing stabilized zirconia powder contains both silver having an average particle diameter of 0.5 $\mu$m or less and silver oxide having an average particle diameter of 0.5 $\mu$m or less, or a case where the silver-containing stabilized zirconia powder contains only silver having an average particle diameter of 0.5 $\mu$m or less, silver oxide particles are converted into silver particles when thermally decomposed, so a sintered body containing silver (silver particles) having an average particle diameter of 0.5 $\mu$m or less can be obtained when the silver-containing stabilized zirconia powder is sintered.

In particular, according to the configuration of [9], the average particle diameter of silver and/or silver oxide is decreased to 0.5 $\mu$m or less and silver and/or silver oxide is highly dispersed in the stabilized zirconia powder. As a result, the crystal grain size of stabilized zirconia in the zirconia sintered body obtained through sintering can be easily controlled to 0.35 $\mu$m or less, and desired hydrothermal degradation properties and mechanical properties can be attained.

Furthermore, the present invention provides the following.

[10] The silver-containing stabilized zirconia powder according to [9], in which a peak top diameter in pore volume distribution is 30 nm or more and 100 nm or less, a pore volume is 0.25 ml/g or more and 0.46 ml/g or less, and a pore distribution width is 40 nm or more and 120 nm or less in a range of 10 nm or more and 0.5 $\mu$m or less in pore distribution based on a mercury intrusion method.

When the average particle diameter of silver and/or silver oxide is decreased to 0.5 $\mu$m or less and silver and/or silver oxide is highly dispersed in the stabilized zirconia powder as well as the pore distribution of the silver-containing stabilized zirconia powder is controlled (optimized) as described above in [10], the crystal grain size of stabilized zirconia in the zirconia sintered body obtained through sintering can be easily controlled to 0.25 $\mu$m or less and desired hydrothermal degradation properties and mechanical properties can be attained.

Furthermore, the present invention provides the following.

[11] A method for producing a silver-containing stabilized zirconia powder, the method including a step of mixing stabilized zirconia containing zirconia and a stabilizer with silver oxide having an average particle diameter of 0.5 $\mu$m or less.

Silver is excellent in ductility and malleability, so it is difficult to atomize silver by pulverization. Meanwhile, silver oxide, because of its nature, can be atomized by pulverization.

According to the configuration of [10], as silver oxide atomized to have an average particle diameter of 0.5 $\mu$m or less is mixed with stabilized zirconia, it is possible to obtain a zirconia powder containing stabilized zirconia and silver oxide having an average particle diameter of 0.5 $\mu$m or less. Here, the silver oxide particles can be converted into silver particles through thermal decomposition by heating. Therefore, when the silver-containing stabilized zirconia powder obtained by the production method of [10] is sintered, it is possible to obtain a sintered body containing silver (silver particles) having an average particle diameter of 0.5 $\mu$m or less.

Furthermore, the present invention provides the following.

[12] A method for producing a silver-containing zirconia sintered body, the method including:

a step X of compacting the silver-containing stabilized zirconia powder according to [9] or [10] to obtain a green compact; and
a step Y of sintering the green compact under conditions of 1250°C or more and 1500°C or less and 1 hour or more and 5 hours or less after the step X.

According to the configuration of [12], since the silver-containing stabilized zirconia powder according to [9] or [10] is used, it is possible to obtain a sintered body having a high sintered density under sintering conditions at a low temperature of 1250°C or more and 1500°C or less. Since the sintering is carried out at a low temperature, the obtained sintered body is excellent in the resistance to hydrothermal degradation.

EFFECT OF THE INVENTION

According to the present invention, it is possible to provide a silver-containing zirconia sintered body that is excellent in mechanical properties, sinterability, and resistance to hydrothermal degradation. It is also possible to provide a silver-containing stabilized zirconia powder that can be converted into the silver-containing zirconia sintered body by being sintered, and to provide a method for producing the silver-containing stabilized zirconia powder. It is also possible to provide a method for producing the silver-containing zirconia sintered body.

BRIEF DESCRIPTION OF THE DRAWINGS

Fig. 1 is a schematic diagram for explaining a method for producing a silver-containing stabilized zirconia powder according to the present embodiment.
Fig. 2 is a schematic diagram for explaining indentation lengths and crack lengths.

MODE FOR CARRYING OUT THE INVENTION

Hereinafter, embodiments of the present invention will be described. However, the present invention is not limited only to these embodiments. In the present description, zirconia (zirconium oxide) is common one, and contains 10 mass% or less of impure metal compounds including hafnia. In the present specification, the terms "comprise" and "contain" include the concepts of "comprise", "contain", "substantially consist of", and "consist of".

[Silver-containing zirconia sintered body]

Hereinafter, an example of the silver-containing zirconia sintered body according to the present embodiment will be described. However, the silver-containing zirconia sintered body of the present invention is not limited to the following example.
The silver-containing zirconia sintered body according to the present embodiment contains:

silver particles; and
stabilized zirconia containing zirconia and a stabilizer, in which
an average particle diameter of the silver particles is 0.5 μm or less,
an average crystal grain size of the stabilized zirconia is 0.35 μm or less, and
a relative sintered density is 98% or more.

The silver-containing zirconia sintered body contains silver particles. The average particle diameter of the silver particles is 0.5 μm or less. Since the average particle diameter of the silver particles is 0.5 μm or less, segregation of silver particles in the silver-containing zirconia sintered body can be suppressed. As a result, the decrease in sinterability is suppressed. In addition, the decrease in mechanical properties is suppressed. In other words, as the dispersibility of silver particles in the silver-containing zirconia sintered body is improved, densification is possible (it is possible to increase the relative sintered density), and the decrease in mechanical properties of the silver-containing zirconia sintered body is suppressed.
The average particle diameter of the silver particles is preferably 0.4 μm or less. It is more preferable as the average particle diameter of the silver particles is smaller, but is, for example, 0.05 μm or more, 0.1 μm or more, or the like.
The average particle diameter of the silver particles is preferably 0.05 μm or more and 0.5 μm or less, more preferably 0.1 μm or more and 0.4 μm or less.
The average particle diameter of the silver particles is measured by the method described in Examples.
The content of the silver particles is preferably in a range of 0.025% by mass or more and 20% by mass or less in

terms of $Ag_2O$ when the sum of zirconia and the stabilizer is taken as 100% by mass. When the content of the silver particles is 0.025% by mass or more, the silver-containing zirconia sintered body is excellent in antibacterial properties. When the content of the silver particles is 20% by mass or less, the silver-containing zirconia sintered body is superior in mechanical properties and sinterability.

The content of the silver particles is more preferably 0.05% by mass or more, still more preferably 0.1% by mass or more, particularly preferably 0.5% by mass or more in terms of $Ag_2O$ when the sum of zirconia and the stabilizer is taken as 100% by mass.

The content of the silver particles is more preferably 16% by mass or less, still more preferably 12% by mass or less, particularly preferably 10% by mass or less in terms of $Ag_2O$ when the sum of zirconia and the stabilizer is taken as 100% by mass.

The content of the silver particles is more preferably 0.05% by mass or more and 16% by mass or less, still more preferably 0.1% by mass or more and 12% by mass or less, particularly preferably 0.5% by mass or more and 10% by mass or less in terms of $Ag_2O$ when the sum of zirconia and the stabilizer is taken as 100% by mass.

The silver-containing zirconia sintered body contains stabilized zirconia containing zirconia and a stabilizer.

The total content of zirconia and the stabilizer (content of stabilized zirconia) in the silver-containing zirconia sintered body is preferably 70% by mass or more, more preferably 80% by mass or more when the entire silver-containing zirconia sintered body is taken as 100% by mass. The total content of zirconia and the stabilizer can be set to 99.9% by mass or less, 99% by mass or less, or the like when the entire silver-containing zirconia sintered body is taken as 100% by mass.

The total content of zirconia and the stabilizer in the silver-containing zirconia sintered body is more preferably 70% by mass or more and 99.9% by mass or less, still more preferably 80% by mass or more and 99.9% by mass or less when the entire silver-containing zirconia sintered body is taken as 100% by mass.

The stabilizer is preferably an oxide of one or more elements selected from alkaline earth metals or rare earth elements. The alkaline earth metals refer to Ca, Sr, Ba, and Ra. The rare earth elements refer to Sc, Y, La, Ce, Pr, Nd, Pm, Sm, Eu, Gd, Tb, Dy, Ho, Er, Tm, Yb, and Lu. It is also preferable that the stabilizer is one or more selected from the group consisting of $Y_2O_3$, $CeO_2$, $Er_2O_3$, and CaO among others. When the stabilizer is one or more selected from the group consisting of $Y_2O_3$, $CeO_2$, $Er_2O_3$, and CaO, the silver-containing zirconia sintered body is superior in low temperature sinterability and mechanical strength.

With regard to the content of the stabilizer, when zirconia and the stabilizer are taken as the whole, the content of the stabilizer is preferably 15 mol% or less. When the content of the stabilizer is 15 mol% or less, the silver-containing zirconia sintered body is superior in mechanical strength.

The content of the stabilizer is preferably 1.4 mol% or more, more preferably 1.5 mol% or more, still more preferably 1.6 mol% or more.

The content of the stabilizer is preferably 14 mol% or less, more preferably 13 mol% or less, still more preferably 12.5 mol% or less.

The content of the stabilizer is more preferably 1.4 mol% or more and 14 mol% or less, still more preferably 1.5 mol% or more and 13 mol% or less, particularly preferably 1.6 mol% or more and 12.5 mol% or less.

In a case where $Y_2O_3$ is used as the stabilizer, the content of $Y_2O_3$ is preferably 1.4 mol% or more and 7.5 mol% or less when zirconia and the stabilizer are taken as the whole. The content of $Y_2O_3$ is more preferably 1.5 mol% or more, still more preferably 1.6 mol% or more. The content of $Y_2O_3$ is more preferably 6.5 mol% or less, still more preferably 6 mol%, particularly preferably 5.6 mol% or less, especially preferably 5 mol% or less, extremely preferably 4.5 mol% or less. When the content of $Y_2O_3$ is 1.4 mol% or more and 7.5 mol% or less, the silver-containing zirconia sintered body is superior in mechanical strength.

In a case where $Y_2O_3$ is used as the stabilizer, the content of $Y_2O_3$ is more preferably 1.5 mol% or more and 6.5 mol% or less, still more preferably 1.6 mol% or more and 6 mol% or less when zirconia and the stabilizer are taken as the whole.

In a case where $CeO_2$ is used as the stabilizer, the content of $CeO_2$ is preferably 10 mol% or more and 15 mol% or less when zirconia and the stabilizer are taken as the whole. The content of $CeO_2$ is more preferably 11 mol% or more, still more preferably 11.5 mol% or more. The content of $CeO_2$ is more preferably 14 mol% or less, still more preferably 13 mol% or less, particularly preferably 12.5 mol% or less, especially preferably 14 mol% or less, extremely preferably 12 mol% or less. When the content of $CeO_2$ is 10 mol% or more and 15 mol% or less, the silver-containing zirconia sintered body is superior in mechanical strength. Among these, the content of $CeO_2$ is preferably 10 mol% or more and 14 mol% or less.

In a case where $CeO_2$ is used as the stabilizer, the content of $CeO_2$ is more preferably 11 mol% or more and 14 mol% or less, still more preferably 11.5 mol% or more and 13 mol% or less, particularly preferably 11.5 mol% or more and 12.5 mol% or less when zirconia and the stabilizer are taken as the whole.

In a case where any one of $Sc_2O_3$, $Er_2O_3$, or $Yb_2O_3$ is used as the stabilizer, the content thereof is preferably 1.4 mol% or more and 7.5 mol% or less when zirconia and the stabilizer are taken as the whole. In a case where any one of $Sc_2O_3$, $Er_2O_3$, or $Yb_2O_3$ is used as the stabilizer, the content thereof is more preferably 1.5 mol% or more, still more preferably 1.6 mol% or more. In a case where any one of $Sc_2O_3$, $Er_2O_3$, or $Yb_2O_3$ is used as the stabilizer, the content thereof is more preferably 6.5 mol% or less, still more preferably 6 mol%, particularly preferably 5.6 mol% or less, especially preferably 5 mol% or less, extremely preferably 4.5 mol% or less. In a case where any one of $Sc_2O_3$, $Er_2O_3$, or $Yb_2O_3$ is used as the stabilizer, when the content thereof is 1.4 mol% or more and 7.5 mol% or less, the silver-containing zirconia sintered body is superior in mechanical strength.

In a case where any one of $Sc_2O_3$, $Er_2O_3$, or $Yb_2O_3$ is used as the stabilizer, the content thereof is more preferably 1.5 mol% or more and 6.5 mol% or less, still more preferably 1.6 mol% or more and 6 mol% or less when zirconia and the stabilizer are taken as the whole.

In a case where CaO is used as the stabilizer, the content of CaO is preferably 3.5 mol% or more and 15 mol% or less when zirconia and the stabilizer are taken as the whole. The content of CaO is more preferably 3.8 mol% or more, still more preferably 4.0 mol% or more. The content of CaO is more preferably 12.0 mol% or less, still more preferably 9.0 mol% or less. When the CaO is preferably 3.5 mol% or more and 15 mol% or less, the silver-containing zirconia sintered body is superior in mechanical strength.

In a case where CaO is used as the stabilizer, the content of CaO is more preferably 3.8 mol% or more and 12 mol% or less, still more preferably 4 mol% or more and 9 mol% or less when zirconia and the stabilizer are taken as the whole.

In a case where CaO and $Y_2O_3$ are used as the stabilizer, the total amount of the stabilizers is 2.5 mol% or more and 6.5 mol% or less in terms of oxide when zirconia and the stabilizers are taken as the whole, and the ratio of [amount (mol%) of CaO]/[total amount (mol%) of stabilizers] is preferably 50% or more and 98% or less.

The ratio of [amount (mol%) of CaO]/[total amount (mol%) of stabilizers] is more preferably 55% or more, still more preferably 60% or more.

The ratio of [amount (mol%) of CaO]/[total amount (mol%) of stabilizers] is more preferably 90% or less, still more preferably 80% or less.

The ratio of [amount (mol%) of CaO]/[total amount (mol%) of stabilizers] is more preferably 55% or more and 90% or less, still more preferably 60% or more and 80% or less.

In a case where CaO and $Y_2O_3$ are used as the stabilizer, the total amount of the stabilizers is preferably 2.5 mol% or more and 6.5 mol% or less in terms of oxide when zirconia and the stabilizers are taken as the whole. When the total amount of the stabilizers is 2.5 mol% or more in terms of oxide, the monoclinic fraction in the obtained zirconia sintered body can be reduced, and it is possible to prevent cracks from being generated in the zirconia sintered body obtained by sintering the zirconia powder. When the total amount of the stabilizers is 6.5 mol% or less in terms of oxide, the fraction of a cubic phase having low mechanical properties (strength, toughness) can be reduced and the fraction of a tetragonal phase having high mechanical properties can be increased.

The total amount of the stabilizers is preferably 2.7 mol% or more, more preferably 2.9 mol% or more, still more preferably 3.0 mol% or more in terms of oxide.

The total amount of the stabilizers is preferably 6.0 mol% or less, more preferably 5.5 mol% or less, still more preferably 5.0 mol% or less, particularly preferably 4.5 mol% or less in terms of oxide.

The total amount of the stabilizers is preferably 2.7 mol% or more and 6.0 mol% or less, more preferably 2.9 mol% or more and 5.5 mol% or less, still more preferably 3.0 mol% or more and 5.0 mol% or less, particularly preferably 3.0 mol% or more and 4.5 mol% or less in terms of oxide.

The average crystal grain size of stabilized zirconia is 0.35 $\mu$m or less. Since the average crystal grain size of stabilized zirconia is 0.35 $\mu$m or less, the silver-containing zirconia sintered body is excellent in the resistance to hydrothermal degradation. When the sintering temperature is set to a high temperature, the crystal grain size of stabilized zirconia increases, but the average crystal grain size of stabilized zirconia is 0.35 $\mu$m or less, and it can be said that the sintering temperature is low.

The average crystal grain size of stabilized zirconia is preferably 0.3 $\mu$m or less, more preferably 0.25 $\mu$m or less, still more preferably 0.2 $\mu$m or less. It is more preferable as the average crystal grain size of stabilized zirconia is smaller, but is, for example, 0.05 $\mu$m or more, 0.1 $\mu$m or more, or the like.

The average crystal grain size of stabilized zirconia is preferably 0.05 $\mu$m or more and 0.3 $\mu$m or less, more preferably 0.05 $\mu$m or more and 0.25 $\mu$m or less. The average crystal grain size of stabilized zirconia is measured by the method described in Examples.

The silver-containing zirconia sintered body may contain $Al_2O_3$ (alumina) in a range of 25% by mass or less when the sum of zirconia and the stabilizer is taken as 100% by mass. When alumina is contained in the range of 25% by mass or less, alumina acts as a sintering aid when zirconia powder is sintered to obtain the silver-containing zirconia sintered body.

In a case where the silver-containing zirconia sintered body contains $Al_2O_3$, from the viewpoint of suitably functioning as a sintering aid, the content of $Al_2O_3$ is more preferably 10% by mass or less, still more preferably 1% by mass or less.

In a case where the silver-containing zirconia sintered body contains $Al_2O_3$, from the viewpoint of suitably functioning as a sintering aid, the content of $Al_2O_3$ is more preferably 0.1% by mass or more, still more preferably 0.25% by mass or more.

In a case where the silver-containing zirconia sintered body contains $Al_2O_3$, the content of $Al_2O_3$ is more preferably 0.1% by mass or more and 10% by mass or less, still more preferably 0.25% by mass or more and 1% by mass or less.

In addition to alumina, the silver-containing zirconia sintered body may contain sinterable ceramics, a thermosetting resin, or the like for the purpose of improving properties such as strength.

The silver-containing zirconia sintered body may contain one or more selected from the group consisting of Fe, V, Mn, Co, Zn, Cu, and Ti. When one or more selected from the group consisting of Fe, V, Mn, Co, Zn, Cu, and Ti are contained, the silver-containing zirconia sintered body can suitably be colored.

<Monoclinic fraction before hydrothermal treatment>

The fraction of the monoclinic phase (monoclinic fraction before hydrothermal treatment) contained in the crystal phase of the silver-containing zirconia sintered body is preferably 0% or more and 10% or less. The monoclinic fraction is preferably 0%, but may be 0.1% or more, 0.5% or more, or the like. The monoclinic fraction is more preferably 5% or less, still more preferably 1% or less.

The monoclinic fraction is more preferably 0% or more and 10% or less, still more preferably 0% or more and 1% or less.

When the monoclinic fraction is 0% or more and 10% or less, the silver-containing zirconia sintered body is superior in mechanical properties (flexural strength, fracture toughness value).

The monoclinic fraction can be controlled by, for example, the average particle diameter of silver particles, the content of silver particles, the content of stabilizer, the content ratio, and the sintering temperature.

The monoclinic fraction is determined by the method described in Examples.

<Resistance to hydrothermal degradation>

The silver-containing zirconia sintered body preferably has a monoclinic fraction of 80% or less after hydrothermal treatment at 134°C and 0.3 MPa (absolute pressure 0.3 MPa) for 15 hours. When the monoclinic fraction after hydrothermal treatment is 80% or less, it can be said that the silver-containing zirconia sintered body is superior in resistance to hydrothermal degradation. The monoclinic fraction after hydrothermal treatment can be controlled by, for example, the average particle diameter of silver particles, the content of silver particles, the content of stabilizer, the content ratio, and the sintering temperature.

The monoclinic fraction after hydrothermal treatment is more preferably 50% or less, still more preferably 10% or less. It is more preferable as the monoclinic fraction after hydrothermal treatment is lower and is, for example, 0.1% or more or 0.5% or more.

The monoclinic fraction after hydrothermal treatment is more preferably 0% or more and 50% or less, still more preferably 0% or more and 10% or less.

<Mechanical strength>

The silver-containing zirconia sintered body preferably has a three-point bending strength of 600 MPa or more.

The three-point bending strength is more preferably 700 MPa or more, still more preferably 800 MPa or more. It is more preferable as the three-point bending strength is higher, but can be set to, for example, 1500 MPa or less or 1200 MPa or less.

The three-point bending strength is more preferably 700 MPa or more and 1500 MPa or less, still more preferably 800 MPa or more and 1500 MPa or less.

When the three-point bending strength is 600 MPa or more, it can be said that the silver-containing zirconia sintered body has higher strength. The three-point bending strength is determined by the method described in Examples.

<Toughness>

The silver-containing zirconia sintered body preferably has a toughness value of 5 $MPa·m^{0.5}$ or more as measured by an IF method.

The toughness value is more preferably 6 $MPa·m^{0.5}$ or more, still more preferably 10 $MPa·m^{0.5}$ or more. It is more preferable as the toughness value is larger, but can be set to, for example, 20 $MPa·m^{0.5}$ or less or 25 $MPa·m^{0.5}$ or less.

The toughness value is more preferably 6 $MPa·m^{0.5}$ or more and 20 $MPa·m^{0.5}$ or less, still more preferably 10 $MPa·m^{0.5}$ or more and 20 $MPa·m^{0.5}$ or less.

When the toughness value is 5 $MPa·m^{0.5}$ or more, it can be said that the silver-containing zirconia sintered body has higher toughness. The toughness value is determined by the method described in Examples.

<Relative sintered density>

The relative sintered density of the silver-containing zirconia sintered body is preferably 98% or more, more preferably 98.5% or more. When the relative sintered density is 98% or more, it can be said that the silver-containing zirconia sintered body has sufficiently been sintered. When the relative sintered density is 98% or more, it can be said that the silver-containing zirconia sintered body has higher strength.

<Method for measuring relative sintered density of silver-containing zirconia sintered body>

The relative sintered density refers to a relative sintered density represented by the following Formula (1).

Relative sintered density (%) = (sintered density/theoretical sintered density) × 100　　　　　　(1)

Here, the theoretical sintered density (denoted by $\rho_0$) is a value calculated by the following Formula (2-1).

$\rho_0$ = (100 + Y + Z)/[(100/$\rho$z) + (Y/3.987) + (Z/10.49)]　　　　　　(2-1)

where Y is the alumina concentration (% by weight) and Z is the silver concentration (% by weight). In a case where silver oxide is used as a raw material, the silver concentration (% by weight) is calculated by Formula (2-2).

Z = [107.868 × (silver oxide concentration (% by weight))]/(231.736 × 2)　　　　　　(2-2)

in which $\rho$z is a value calculated by the following Formula (2-3).

$\rho$z = [124.25(100 - X) + [molecular weight of stabilizer] × X]/150[150.5(100 + X)$A^2$C　　　　　　(2-3)

Here, as the molecular weight of the stabilizer, 225.81 is used when the stabilizer is $Y_2O_3$, 382.52 is used when the stabilizer is $Er_2O_3$, and 394.11 is used when the stabilizer is $Yb_2O_3$.
X is the stabilizer concentration (mol%). A and C are values calculated by the following Formulas (2-4) and (2-5), respectively.

```
A = 0.5080 + 0.06980X/(100 + X) ... (2-4)

C = 0.5195 - 0.06180X/(100 + X) ... (2-5)
```

In Formula (1), the theoretical sintered density varies depending on the composition of the powder.
For example, the theoretical sintered density of yttria-containing zirconia is 6.117 g/cm$^3$ when the yttria content is 2 mol%, 6.098 g/cm$^3$ when the yttria content is 3 mol%, and 6.051 g/cm$^3$ when the yttria content is 5.5 mol% (in the case of $Al_2O_3$ = 0% by weight).
When the stabilizer is $Sc_2O_3$, $\rho$z is a value calculated by the following Formula (3).

$\rho$z = -0.0402 (molar concentration of $Sc_2O_3$) + 6.1294　　　　　　(3)

When the stabilizer is CaO, $\rho$z is a value calculated by the following Formula (3-1).

```
ρz = -0.0400(molar concentration of CaO) + 6.1700 ...

(3-1)
```

The theoretical sintered density (denoted by $\rho$1) in the case of containing a colorant is

```
ρ1 = 100/[(Z/V) + (100 - Z)/ρ0] ... (2-5)
```

Z is the concentration (% by weight) of the colorant, and V is the theoretical density (g/cm$^3$) of the colorant.

The theoretical density of the colorant is 5.24 g/cm$^3$ for $Fe_2O_3$, 5.61 g/cm$^3$ for ZnO, 5.03 g/cm$^3$ for $MnO_2$, 6.10 g/cm$^3$ for CoO, 5.22 g/cm$^3$ for $Cr_2O_3$, 4.23 g/cm$^3$ for $TiO_2$, 7.80 g/cm$^3$ for $Tb_4O_7$, 6.31 g/cm$^3$ for CuO, and 3.36 g/cm$^3$ for $V_2O_5$.

The sintered density is measured by Archimedes method.

The silver-containing zirconia sintered body according to the present embodiment can be obtained by, for example, a method for producing a silver-containing zirconia sintered body described later.

The silver-containing zirconia sintered body according to the present embodiment can be used as an industrial part, an aesthetic part, or a dental material. More specifically, the silver-containing zirconia sintered body can be used for jewelry, watch parts, watch faces, artificial teeth, members for molding processing, wear resistant members, chemical resistant members, and the like.

In particular, when a blade is produced using the silver-containing zirconia sintered body, the decrease in antibacterial action caused by wear during use and sharpening of the blade can be suppressed. Since the resistance to hydrothermal degradation is improved by low temperature sintering, the silver-containing zirconia sintered body can exert stable mechanical properties even under conditions that cause hydrothermal degradation, such as in a dishwasher.

[Silver-containing stabilized zirconia powder]

The silver-containing stabilized zirconia powder according to the present embodiment contains:

stabilized zirconia containing zirconia and a stabilizer; and
silver and/or silver oxide having an average particle diameter of 0.5 μm or less.

The silver-containing stabilized zirconia powder contains primary particles of stabilized zirconia containing zirconia as a main component. All or some of the primary particles are aggregated to form secondary particles. In other words, the silver-containing stabilized zirconia powder contains primary particles that are not aggregated and secondary particles in which primary particles are aggregated.

However, in the silver-containing stabilized zirconia powder, the number of primary particles that do not form secondary particles and are present in the state of unaggregated primary particles is very small and is, for example, less than 1% by mass of the entire primary particles (the sum of unaggregated primary particles and primary particles aggregated and forming secondary particles). In other words, the silver-containing stabilized zirconia powder may contain a very small amount of unaggregated primary particles, but most of the silver-containing stabilized zirconia powder is composed of secondary particles.

The phrase "containing zirconia as a main component" means that the primary particles contain zirconia at 70% by mass or more when the primary particles are taken as 100% by mass. In other words, in the present description, primary particles containing zirconia as a main component refer to primary particles containing zirconia at 70% by mass or more. The content of zirconia contained in the primary particles is preferably 74% by mass or more, more preferably 80% by mass or more, still more preferably 85% by mass or more.

<Composition>

The silver-containing stabilized zirconia powder according to the present embodiment contains silver and/or silver oxide having an average particle diameter of 0.5 μm or less.

Silver oxide particles are converted into silver particles when thermally decomposed. According to the silver-containing stabilized zirconia powder, since stabilized zirconia and silver and/or silver oxide having an average particle diameter of 0.5 μm or less are contained, a sintered body containing silver (silver particles) having an average particle diameter of 0.5 μm or less can be obtained when the silver-containing stabilized zirconia powder is sintered.

In other words, in any one of a case where the silver-containing stabilized zirconia powder contains only silver oxide having an average particle diameter of 0.5 μm or less, a case where the silver-containing stabilized zirconia powder contains both silver having an average particle diameter of 0.5 μm or less and silver oxide having an average particle diameter of 0.5 μm or less, or a case where the silver-containing stabilized zirconia powder contains only silver having an average particle diameter of 0.5 μm or less, silver oxide particles are converted into silver particles when thermally decomposed, so a sintered body containing silver (silver particles) having an average particle diameter of 0.5 μm or less can be obtained when the silver-containing stabilized zirconia powder is sintered.

In the present description, the average particle diameter of silver and/or silver oxide refers to the average particle diameter of the total of silver and silver oxide, without distinguishing silver and silver oxide from each other. The average particle diameter of silver and/or silver oxide is measured by the method described in Examples.

The average particle diameter of silver and/or silver oxide is preferably 0.4 μm or less, more preferably 0.3 μm or

less. It is more preferable as the average particle diameter of silver and/or silver oxide is smaller, but is, for example, 0.01 $\mu$m or more, 0.05 $\mu$m or more, or the like.

The average particle diameter of silver and/or silver oxide is preferably 0.01 $\mu$m or more and 0.4 $\mu$m or less, more preferably 0.01 $\mu$m or more and 0.3 $\mu$m or less.

The content of silver and/or silver oxide is preferably in a range of 0.025% by mass or more and 20% by mass or less in terms of $Ag_2O$ when the sum of zirconia and the stabilizer is taken as 100% by mass. When the content of silver and/or silver oxide is 0.025% by mass or more, the silver-containing stabilized zirconia powder is excellent in antibacterial properties. When the content of silver and/or silver oxide is 20% by mass or less, the silver-containing stabilized zirconia powder is superior in sinterability. When the content of silver and/or silver oxide is 20% by mass or less, the mechanical properties of the obtained sintered body are superior.

The content of silver and/or silver oxide is more preferably 0.05% by mass or more, still more preferably 0.1% by mass or more, particularly preferably 0.5% by mass or more in terms of $Ag_2O$ when the sum of zirconia and the stabilizer is taken as 100% by mass.

The content of silver and/or silver oxide is more preferably 16% by mass or less, still more preferably 12% by mass or less, particularly preferably 10% by mass or less in terms of $Ag_2O$ when the sum of zirconia and the stabilizer is taken as 100% by mass.

The content of silver and/or silver oxide is more preferably 0.05% by mass or more and 16% by mass or less, still more preferably 0.1% by mass or more and 12% by mass or less, particularly preferably 0.5% by mass or more and 10% by mass or less in terms of $Ag_2O$ when the sum of zirconia and the stabilizer is taken as 100% by mass.

The silver-containing stabilized zirconia powder contains stabilized zirconia containing zirconia and a stabilizer.

The stabilized zirconia comprises a stabilizer. The stabilizer is contained in the primary particles in a form of solid-dissolving or the like. Since the stabilized zirconia comprises the stabilizer, the silver-containing stabilized zirconia powder can be suitably sintered at a low temperature.

The stabilizer is preferably an oxide of one or more elements selected from alkaline earth metals or rare earth elements. The alkaline earth metals refer to Ca, Sr, Ba, and Ra. The rare earth elements refer to Sc, Y, La, Ce, Pr, Nd, Pm, Sm, Eu, Gd, Tb, Dy, Ho, Er, Tm, Yb, and Lu. It is also preferable that the stabilizer is one or more selected from the group consisting of $Y_2O_3$, $CeO_2$, $Er_2O_3$, and CaO among others. When the stabilizer is one or more selected from the group consisting of $Y_2O_3$, $CeO_2$, $Er_2O_3$, and CaO, the silver-containing stabilized zirconia powder is superior in low temperature sinterability and the mechanical strength of the obtained sintered body is superior.

With regard to the content of the stabilizer, when zirconia and the stabilizer are taken as the whole, the content of the stabilizer is preferably 15 mol% or less. When the content of the stabilizer is 15 mol% or less, the mechanical strength of the obtained sintered body is superior.

The content of the stabilizer is preferably 1.4 mol% or more, more preferably 1.5 mol% or more, still more preferably 1.6 mol% or more.

The content of the stabilizer is preferably 14 mol% or less, more preferably 13 mol% or less, still more preferably 12.5 mol% or less.

The content of the stabilizer is more preferably 1.4 mol% or more and 14 mol% or less, still more preferably 1.5 mol% or more and 13 mol% or less, particularly preferably 1.6 mol% or more and 12.5 mol% or less.

In a case where $Y_2O_3$ is used as the stabilizer, the content of $Y_2O_3$ is preferably 1.4 mol% or more and 7.5 mol% or less when zirconia and the stabilizer are taken as the whole. The content of $Y_2O_3$ is more preferably 1.5 mol% or more, still more preferably 1.6 mol% or more. The content of $Y_2O_3$ is more preferably 6.5 mol% or less, still more preferably 6 mol%, particularly preferably 5.6 mol% or less, especially preferably 5 mol% or less, extremely preferably 4.5 mol% or less. When the content of $Y_2O_3$ is 1.4 mol% or more and 7.5 mol% or less, a zirconia sintered body obtained by sintering the silver-containing stabilized zirconia powder is superior in mechanical strength.

In a case where $Y_2O_3$ is used as the stabilizer, the content of $Y_2O_3$ is more preferably 1.5 mol% or more and 6.5 mol% or less, still more preferably 1.6 mol% or more and 6 mol% or less when zirconia and the stabilizer are taken as the whole.

In a case where $CeO_2$ is used as the stabilizer, the content of $CeO_2$ is preferably 10 mol% or more and 15 mol% or less when zirconia and the stabilizer are taken as the whole. The content of $CeO_2$ is more preferably 11 mol% or more, still more preferably 11.5 mol% or more. The content of $CeO_2$ is more preferably 14 mol% or less, still more preferably 13 mol% or less, particularly preferably 12.5 mol% or less, especially preferably 12 mol% or less. When the content of $CeO_2$ is 10 mol% or more and 15 mol% or less, a zirconia sintered body obtained by sintering the silver-containing stabilized zirconia powder is superior in mechanical strength. Among these, the content of $CeO_2$ is preferably 10 mol% or more and 14 mol% or less.

In a case where $CeO_2$ is used as the stabilizer, the content of $CeO_2$ is more preferably 11 mol% or more and 14 mol% or less, still more preferably 11.5 mol% or more and 13 mol% or less, particularly preferably 11.5 mol% or more and 12.5 mol% or less when zirconia and the stabilizer are taken as the whole.

In a case where any one of $Sc_2O_3$, $Er_2O_3$, or $Yb_2O_3$ is used as the stabilizer, the content thereof is preferably 1.4 mol% or more and 7.5 mol% or less when zirconia and the stabilizer are taken as the whole. In a case where any one of $Sc_2O_3$, $Er_2O_3$, or $Yb_2O_3$ is used as the stabilizer, the content thereof is more preferably 1.5 mol% or more, still more preferably 1.6 mol% or more. In a case where any one of $Sc_2O_3$, $Er_2O_3$, or $Yb_2O_3$ is used as the stabilizer, the content thereof is more preferably 6.5 mol% or less, still more preferably 6 mol%, particularly preferably 5.6 mol% or less, especially preferably 5 mol% or less, extremely preferably 4.5 mol% or less. In a case where any one of $Sc_2O_3$, $Er_2O_3$, or $Yb_2O_3$ is used as the stabilizer, when the content thereof is 1.4 mol% or more and 7.5 mol% or less, a zirconia sintered body obtained by sintering the silver-containing stabilized zirconia powder is superior in mechanical strength.

In a case where any one of $Sc_2O_3$, $Er_2O_3$, or $Yb_2O_3$ is used as the stabilizer, the content thereof is more preferably 1.5 mol% or more and 6.5 mol% or less, still more preferably 1.6 mol% or more and 6 mol% or less when zirconia and the stabilizer are taken as the whole.

In a case where CaO is used as the stabilizer, the content of CaO is preferably 3.5 mol% or more and 15 mol% or less when zirconia and the stabilizer are taken as the whole. The content of CaO is more preferably 3.8 mol% or more, still more preferably 4.0 mol% or more. The content of CaO is more preferably 12.0 mol% or less, still more preferably 9.0 mol% or less. When the content of CaO is 3.5 mol% or more and 15 mol% or less, a zirconia sintered body obtained by sintering the silver-containing stabilized zirconia powder is superior in mechanical strength.

In a case where CaO is used as the stabilizer, the content of CaO is more preferably 3.8 mol% or more and 12 mol% or less, still more preferably 4 mol% or more and 9 mol% or less when zirconia and the stabilizer are taken as the whole.

In a case where CaO and $Y_2O_3$ are used as the stabilizer, the total amount of the stabilizers is 2.5 mol% or more and 6.5 mol% or less in terms of oxide when zirconia and the stabilizers are taken as the whole, and the ratio of [amount (mol%) of CaO]/[total amount (mol%) of stabilizers] is preferably 50% or more and 98% or less.

The ratio of [amount (mol%) of CaO]/[total amount (mol%) of stabilizers] is more preferably 55% or more, still more preferably 60% or more.

The ratio of [amount (mol%) of CaO]/[total amount (mol%) of stabilizers] is more preferably 90% or less, still more preferably 80% or less.

The ratio of [amount (mol%) of CaO]/[total amount (mol%) of stabilizers] is more preferably 55% or more and 90% or less, still more preferably 60% or more and 80% or less.

In a case where CaO and $Y_2O_3$ are used as the stabilizer, the total amount of the stabilizers is preferably 2.5 mol% or more and 6.5 mol% or less in terms of oxide when zirconia and the stabilizers are taken as the whole. When the total amount of the stabilizers is 2.5 mol% or more in terms of oxide, the monoclinic fraction in the obtained zirconia sintered body can be reduced, and it is possible to prevent cracks from being generated in the zirconia sintered body obtained by sintering the zirconia powder. When the total amount of the stabilizers is 6.5 mol% or less in terms of oxide, the fraction of a cubic phase having low mechanical properties (strength, toughness) can be reduced and the fraction of a tetragonal phase having high mechanical properties can be increased.

The total amount of the stabilizers is preferably 2.7 mol% or more, more preferably 2.9 mol% or more, still more preferably 3.0 mol% or more in terms of oxide.

The total amount of the stabilizers is preferably 6.0 mol% or less, more preferably 5.5 mol% or less, still more preferably 5.0 mol% or less, particularly preferably 4.5 mol% or less in terms of oxide.

The total amount of the stabilizers is preferably 2.7 mol% or more and 6.0 mol% or less, more preferably 2.9 mol% or more and 5.5 mol% or less, still more preferably 3.0 mol% or more and 5.0 mol% or less, particularly preferably 3.0 mol% or more and 4.5 mol% or less in terms of oxide.

The silver-containing zirconia powder may contain $Al_2O_3$ (alumina) in a range of 25% by mass or less when the sum of zirconia and the stabilizer is taken as 100% by mass. When alumina is contained in the range of 25% by mass or less, alumina acts as a sintering aid when the zirconia powder is sintered to obtain a silver-containing zirconia sintered body.

In a case where the silver-containing zirconia powder contains $Al_2O_3$, from the viewpoint of suitably functioning as a sintering aid, the content of $Al_2O_3$ is more preferably 10% by mass or less, still more preferably 1% by mass or less.

In a case where the silver-containing zirconia powder contains $Al_2O_3$, from the viewpoint of suitably functioning as a sintering aid, the content of $Al_2O_3$ is more preferably 0.1% by mass or less, still more preferably 0.25% by mass or less.

In a case where the silver-containing stabilized zirconia sintered body contains $Al_2O_3$, the content of $Al_2O_3$ is more preferably 0.1% by mass or more and 10% by mass or less, still more preferably 0.25% by mass or more and 1% by mass or less.

In a case where the form of alumina in the silver-containing stabilized zirconia powder is a powder, the average particle diameter of primary particles of alumina is not particularly limited but is, for example, 0.02 to 0.4 $\mu$m, preferably 0.05 to 0.3 $\mu$m, more preferably 0.07 to 0.2 $\mu$m.

The silver-containing stabilized zirconia powder can be sintered at a low temperature in a case where the silver-containing stabilized zirconia powder has the following pore volume distribution. Therefore, even if a configuration in which a sintering aid is not contained is adopted, low temperature sintering can be performed.

The silver-containing stabilized zirconia powder may comprise sinterable ceramics, a thermosetting resin, or the like in addition to alumina for the purpose of improving such characteristics as strength.

The silver-containing stabilized zirconia powder may contain one or more elements selected from the group consisting of Fe, V, Mn, Co, Cr, Tb, Zn, Cu, and Ti. When the silver-containing stabilized zirconia powder contains one or more elements selected from the group consisting of Fe, V, Mn, Co, Cr, Tb, Zn, Cu, and Ti as a coloring element, a zirconia sintered body obtained by sintering the zirconia powder can be suitably colored.

<Pore distribution>

1. Peak top diameter of interparticle spaces of primary particles

The silver-containing stabilized zirconia powder preferably has a peak top diameter of 20 nm or more and 200 nm or less in a pore volume distribution in a range of 10 nm or more and 0.5 $\mu$m or less in a pore distribution based on a mercury intrusion method. The peak top diameter is preferably 25 nm or more, more preferably 30 nm or more, still more preferably 32 nm or more, and particularly preferably 35 nm or more. The peak top diameter is preferably 150 nm or less, more preferably 120 nm or less, still more preferably 100 nm or less, and particularly preferably 85 nm or less.

The peak top diameter is preferably 25 nm or more and 150 nm or less, and more preferably 30 nm or more and 120 nm or less.

When there are a plurality of peaks in the range of 10 nm or more and 0.5 $\mu$m or less in the pore distribution, the phrase "the peak top diameter is 20 nm or more and 200 nm or less in the pore volume distribution" as used herein means that all the peak top diameters in the range of 10 nm or more and 0.5 $\mu$m or less in the pore distribution are in the range of 20 nm or more and 200 nm or less.

2. Pore distribution width of interparticle spaces of primary particles

The silver-containing stabilized zirconia powder preferably has a pore distribution width of 40 nm or more and 200 nm or less in a range of 10 nm or more and 0.5 $\mu$m or less in a pore distribution based on a mercury intrusion method. The pore distribution width is preferably 43 nm or more, more preferably 46 nm or more, still more preferably 50 nm or more, and particularly preferably 55 nm or more. The pore distribution width is preferably 150 nm or less, more preferably 120 nm or less, still more preferably 110 nm or less, particularly preferably 100 nm or less, and especially preferably 80 nm or less.

The pore distribution width is preferably 43 nm or more and 150 nm or less, more preferably 46 nm or more, and 120 nm or less.

Here, the pore distribution width refers to a width of a peak at which the log differential pore volume is 0.1 ml/g or more.

In the case where there are a plurality of peaks in the range of 10 nm or more and 0.5 $\mu$m or less in the pore distribution, the phrase "a pore distribution width is 40 nm or more and 200 nm or less" as used herein means that where, in a graph showing a pore distribution with pore diameter as abscissa against log differential pore volume as ordinate, a point intersecting with a log differential pore volume of 0.1 mL/g for the first time as viewed from a side where the pore diameter is smaller (a point intersecting while ascending) is defined as a minimum diameter and a point intersecting with the log differential pore volume of 0.1 mL/g again (a point intersecting while descending) is defined as a maximum diameter, the difference between the maximum diameter and the minimum diameter is 40 nm or more and 200 nm or less.

3. Pore volume of interparticle spaces of primary particles

The silver-containing stabilized zirconia powder preferably has a pore volume of 0.2 ml/g or more and less than 0.5 ml/g in a range of 10 nm or more and 0.5 $\mu$m or less in a pore distribution based on a mercury intrusion method. The pore volume is preferably 0.22 ml/g or more, more preferably 0.25 ml/g or more, still more preferably 0.3 ml/g or more, particularly preferably 0.35 ml/g or more, and especially preferably 0.4 ml/g or more. The total pore volume is preferably 0.48 ml/g or less, more preferably 0.46 ml/g or less, and particularly preferably 0.44 ml/g or less.

The pore volume is preferably 0.22 ml/g or more and 0.48 ml/g or less, more preferably 0.25 ml/g or more and 0.46 ml/g or less

The "range of 10 nm or more and 0.5 $\mu$m or less in the pore distribution based on the mercury intrusion method" is a range in which interparticle spaces of primary particles of the silver-containing stabilized zirconia powder can be present.

In the range of 10 nm or more and 0.5 $\mu$m or less in the pore distribution based on the mercury intrusion method, when the peak top diameter in the pore volume distribution is 20 nm or more and 200 nm or less and the pore distribution width is 40 nm or more and 200 nm or less, the respective pores (interparticle spaces of primary particles) are small and uniform in size (the distribution is sharp).

Accordingly, the respective primary particles constituting the secondary particles are uniformly and densely aggregated, and there are no large pores.

Here, zirconia particles (including primary particles and secondary particles) have a characteristic that the particles are less likely to be sintered as the pore volume is large. In other words, in order to sinter zirconia particles at a low temperature, it is necessary not only to reduce the size of pores derived from interparticle spaces of primary particles in secondary particles and sharpen the distribution of the pores, but also to simultaneously reduce the volume of the pores derived from the interparticle spaces of the primary particles.

Hence, when the pore volume in the range of 10 nm or more and 0.5 $\mu$m or less in the pore distribution based on the mercury intrusion method is 0.2 ml/g or more and less than 0.5 ml/g, a configuration, in which the volume of pores derived from the interparticle spaces of primary particles is small and large pores are not present, is achieved, and it is possible to obtain a sintered body having a high sintered density.

As described above, by controlling the pore diameter, the pore distribution, and the pore volume of the interparticle spaces of primary particles, it is possible to sinter the silver-containing stabilized zirconia powder at a low temperature and to obtain a sintered body having a high sintered density.

<Particle diameter $D_{50}$>

The particle diameter $D_{50}$ of the silver-containing stabilized zirconia powder is preferably 0.1 $\mu$m or more and 1.5 $\mu$m or less. The particle diameter $D_{50}$ is preferably 0.1 $\mu$m or more, more preferably 0.20 $\mu$m or more, still more preferably 0.25 $\mu$m or more, particularly preferably 0.30 $\mu$m or more. The particle diameter $D_{50}$ is preferably 1.2 $\mu$m or less, more preferably 1.0 $\mu$m or less, still more preferably 0.8 $\mu$m or less, particularly preferably 0.7 $\mu$m or less, especially preferably 0.6 $\mu$m or less, extremely preferably less than 0.5 $\mu$m.

The particle diameter $D_{50}$ of the silver-containing stabilized zirconia powder is preferably 0.1 $\mu$m or more and 1.5 $\mu$m or less, more preferably 0.20 $\mu$m or more and 1.0 $\mu$m or less.

The particle diameter $D_{50}$ refers to a value acquired by the method described in Examples.

When the particle diameter $D_{50}$ is measured, not only secondary particles but also unaggregated primary particles may be contained, but the amount of unaggregated primary particles that may be contained in the silver-containing stabilized zirconia powder is very small. Therefore, the particle diameter $D_{50}$ may be regarded as representing the particle diameter $D_{50}$ of secondary particles, that is, the average particle diameter of secondary particles.

When the particle diameter $D_{50}$ of the silver-containing stabilized zirconia powder is 1.5 $\mu$m or less, the particle diameter of secondary particles is relatively small, and thus interparticle spaces of secondary particles can be reduced. As a result, the silver-containing stabilized zirconia powder is excellent in low temperature sinterability. Since interparticle spaces of secondary particles are small, a sintered body having a high sintered density can be obtained.

<Specific surface area>

The specific surface area of the silver-containing stabilized zirconia powder is preferably 5 m$^2$/g or more and 60 m$^2$/g or less. The specific surface area is preferably 5 m$^2$/g or more, more preferably 9 m$^2$/g or more, still more preferably 13 m$^2$/g or more, and particularly preferably 15 m$^2$/g or more. The specific surface area is preferably 50 m$^2$/g or less, more preferably 40 m$^2$/g or less, still more preferably 35 m$^2$/g or less, and particularly preferably 30 m$^2$/g or less.

The specific surface area is more preferably 9 m$^2$/g or more and 40 m$^2$/g or less, still more preferably 13 m$^2$/g or more and 35 m$^2$/g or less, and particularly preferably 15 m$^2$/g or more and 30 m$^2$/g or less.

In order to sinter a stabilized zirconia powder at about 1300°C to 1500°C, which is lower than the conventional temperature, it is effective to increase the specific surface area. Conventionally, however, it is difficult to control the grain growth rate at the time of producing the stabilized zirconia powder, and it has been considered preferable to set the specific surface area to less than 15 m$^2$/g in order to prevent pores from remaining in the stabilized zirconia powder as much as possible.

Meanwhile, when the interparticle spaces of primary particles are controlled as explained above, a large number of pores do not remain in the silver-containing stabilized zirconia powder even when the specific surface area is 15 m$^2$/g or more. Accordingly, when the specific surface area is 15 m$^2$/g or more, the low-temperature sinterability can be improved.

The specific surface area refers to a value obtained by the method described in Examples.

The silver-containing stabilized zirconia powder according to the present embodiment has been described above.

[Method for producing silver-containing stabilized zirconia powder]

Hereinafter, an example of a method for producing a silver-containing stabilized zirconia powder will be described. However, the method for producing a silver-containing stabilized zirconia powder is not limited to the following examples.

The method for producing a silver-containing stabilized zirconia powder according to the present embodiment includes

a step of mixing stabilized zirconia containing zirconia and a stabilizer with silver oxide having an average particle diameter of 0.5 $\mu$m or less.

The method for producing a silver-containing stabilized zirconia powder preferably includes:

step 1 in which a zirconium salt solution and a sulfating agent solution are separately heated to 95°C or more and 100°C or less;

step 2 in which the heated zirconium salt solution and the heated sulfating agent solution are brought into contact with each other so that the concentration of a mixed liquid does not change from start to end of the contact, thereby obtaining a basic zirconium sulfate-containing reaction liquid as a mixed liquid;

step 3 in which the basic zirconium sulfate-containing reaction liquid obtained in step 2 is aged at 95°C or more for 3 hours or more;

step 4 in which a stabilizer is added to the aged basic zirconium sulfate-containing reaction liquid obtained in step 3;

step 5 in which an alkali is added to the basic zirconium sulfate-containing reaction liquid obtained in step 4 to obtain a zirconium-containing hydroxide;

step 6 in which the zirconium-containing hydroxide obtained in step 5 is subjected to heat treatment to obtain stabilized zirconia; and

step 7 in which stabilized zirconia obtained in step 6 is mixed with silver oxide having an average particle diameter of 0.5 $\mu$m or less, and

in step 2, from start to end of the contact, the weight ratio $SO_4^{2-}/ZrO_2$ in the mixed liquid is maintained in a range of 0.3 to 0.8 as well as the temperature of the mixed liquid is maintained at 95°C or more.

Hereinafter, each of the steps will be described in detail.

<Step 1>

In step 1, a zirconium salt solution and a sulfating agent solution as starting materials are separately heated to 95°C or higher and 100°C or lower.

The zirconium salt to be used for preparing the zirconium salt solution may be any one that supplies zirconium ions, and for example, zirconium oxynitrate, zirconium oxychloride, and zirconium nitrate can be used. One or two or more thereof may be used. Among these, zirconium oxychloride is preferable in terms of its high productivity on an industrial scale.

The solvent to be used for forming the zirconium salt solution may be chosen according to the type, etc. of the zirconium salt. Usually, water (pure water or ion-exchanged water, the same applies hereinafter) is preferable.

The concentration of the zirconium salt solution is not particularly limited, and in general, the zirconium salt is preferably contained in an amount of 5 to 250 g, more preferably 20 to 150 g, in terms of zirconium oxide ($ZrO_2$) based on 1000 g of the solvent.

The sulfating agent may be any one that reacts with zirconium ions to produce a sulfate (that is, a sulfating reagent), and examples thereof include sodium sulfate, potassium sulfate, ammonium sulfate, potassium hydrogen sulfate, sodium hydrogen sulfate, potassium disulfate, sodium disulfate, and sulfur trioxide. The sulfating agent may be in any form such as a powder or solution form, and a solution (especially, an aqueous solution) is preferable. As the solvent, the same solvent as the solvent to be used for preparing the zirconium salt solution can be used.

The acid concentration of the zirconium salt solution is preferably set to 0.1 to 2.0 N. By setting the acid concentration within the above range, the aggregation state of the particles constituting the silver-containing stabilized zirconia powder can be controlled to a suitable state. The acid concentration can be adjusted by using, for example, hydrochloric acid, nitric acid, sodium hydroxide, or the like.

The concentration of the sulfating agent (the sulfating agent solution) is not particularly limited, and in general, it is preferable that the amount of the sulfating agent is 5 to 250 g, particularly 20 to 150 g, based on 1000 g of the solvent.

The containers for preparing the zirconium salt solution and the sulfating agent solution are not particularly limited with respect to their materials as long as the containers each have a capacity large enough for sufficiently stirring the zirconium salt solution and the sulfating agent solution. However, the containers preferably have equipment capable of appropriately heating such that the temperature of each solution does not fall below 95°C.

The heating temperature of the zirconium salt solution and the sulfating agent solution is just required to be 95°C or higher and 100 °C or lower, and is preferably 97°C or higher. When step 2 is performed while the temperature of the zirconium salt solution and the sulfating agent solution is kept lower than 95°C, the zirconium salt solution and the sulfating agent do not sufficiently react with each other, resulting in a lowered yield.

<Step 2>

In step 2, the heated zirconium salt solution and the heated sulfating agent solution are brought into contact with each other such that the concentration of a mixed liquid does not change from the start to the end of the contact, thereby affording a basic zirconium sulfate-containing reaction solution as a mixed liquid. Here, from the start to the end of the contact, the weight ratio $SO_4{}^{2-}/ZrO_2$ in the mixed liquid is maintained in a range of 0.3 to 0.8, and the temperature of the mixed liquid is maintained at 95°C or higher.

Hereinafter, step 2 will be described with reference to drawings.

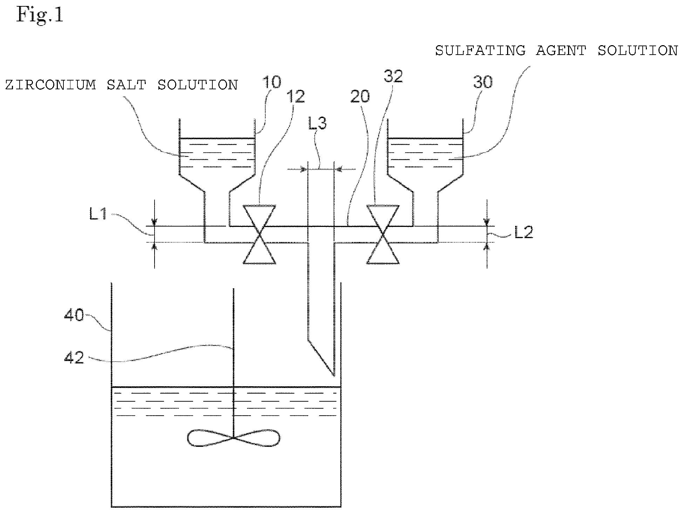

Fig. 1 is a schematic view for explaining a method for producing a silver-containing stabilized zirconia powder according to the present embodiment. As shown in Fig. 1, the container 10 is connected to one end (left side in Fig. 1) above the T-shaped tube 20 via a valve 12. The container 30 is connected to the other end (the right side in Fig. 1) above the T-shaped tube 20 via a valve 32. In the container 10 is stored a zirconium solution heated to 95°C or higher and 100°C or lower. In the container 30 is stored a sulfating agent solution heated to 95°C or higher and 100°C or lower.

In step 2, the valve 12 is opened and the valve 32 is opened to bring the zirconium solution into contact with the sulfating agent solution. The mixed liquid (basic zirconium sulfate-containing reaction liquid) obtained by the contact immediately flows into an aging container 40 from the lower side of the T-shaped tube 20. In step 2, by such a method, the concentration of the reaction liquid (the concentration of the reaction liquid in the T-shaped tube 20) is prevented from changing from the start to the end of the contact of the zirconium solution with the sulfating agent solution. In step 2, since the concentration change of $SO_4{}^{2-}/ZrO_2$ from the start to the end of contact is suppressed, a uniform reactant is obtained. By adopting such a step (step 2), the peak top diameter, the pore volume, and the pore distribution width of primary particles can be controlled. That is, the size of the pores derived from the interparticle spaces of the primary particles in the secondary particles can be reduced, the distribution of the pores can be sharpened, and the volume of pores derived from the interparticle spaces of the primary particles can also be reduced.

The weight ratio $SO_4{}^{2-}/ZrO_2$ in the mixed liquid in step 2 is preferably within a range of 0.3 to 0.8, more preferably 0.4 to 0.7, and still more preferably 0.45 to 0.65. When the weight ratio $SO_4{}^{2-}/ZrO_2$ in the mixed liquid is 0.3 or more, the yield of basic zirconium sulfate as a reaction product can be increased. In addition, by adjusting the $SO_4{}^{2-}/ZrO_2$ weight ratio in the mixed liquid to 0.8 or less, it is possible to suppress the formation of a soluble salt of zirconium sulfate and to suppress a decrease in the yield of basic zirconium sulfate.

In step 2, in order to maintain the temperature of the mixed liquid at 95°C or higher, it is preferable to install a heater in a tube (for example, T-shaped tube 20) or the like for supplying each solution.

Hereinafter, an example of step 2 will be specifically described.

When 213 g of a 25 mass% aqueous sodium sulfate solution and 450 g of an aqueous zirconium oxychloride solution with a concentration of 16 mass% in terms of $ZrO_2$ are brought into contact with each other using a T-shaped tube having a tube diameter L1 of 10 mm at one upper end (the left side in Fig. 1), a tube diameter L2 of 10 mm at the other upper end (the right side in Fig. 1), and a tube diameter L3 of 15 mm at the lower end as the T-shaped tube 20, the time (contact time) from the start of contact to the end of contact (until the aqueous zirconium chloride solution in the container 10 and the sulfating agent solution in the container 30 disappear) is preferably 30 seconds to 300 seconds, more preferably 60 seconds to 200 seconds, and still more preferably 90 seconds to 150 seconds.

<Step 3>

In step 3, the basic zirconium sulfate-containing reaction liquid obtained in step 2 is aged at 95°C or higher for 3 hours or more. In step 3, for example, the basic zirconium sulfate-containing reaction liquid flowing into the aging container 40 is aged at 95°C or higher for 3 hours or more while being stirred with a stirrer 42. The upper limit of the aging time is not particularly limited, and is, for example, 7 hours or less. The temperature (aging temperature) of the mixed liquid (the basic zirconium sulfate-containing reaction liquid) in step 3 is preferably 95°C or higher, and more preferably 97°C or higher and 100°C or lower. By setting the aging temperature to 95°C or higher and the aging time to 3 hours or more, basic zirconium sulfate is sufficiently produced, and the yield can be increased.

The mixed liquid contains basic zirconium sulfate as a main component, and is a basic zirconium sulfate slurry.

<Step 4>

In step 4, a stabilizer is added to the basic zirconium sulfate-containing reaction liquid after aging obtained in step 3.

<Step 5>

In step 5, an alkali is added to the basic zirconium sulfate-containing reaction solution obtained in step 4 to perform a neutralization reaction. Neutralization produces a zirconium-containing hydroxide.

The alkali is not limited, and examples thereof include sodium hydroxide, sodium carbonate, ammonia, and hydrazine ammonium bicarbonate. The alkali is not particularly limited in concentration, and one diluted with water and having a concentration of 5 to 30% is usually used.

While as a method for adding the alkali there are two methods: (1) adding an alkali solution to the basic zirconium sulfate-containing reaction liquid and (2) adding the basic zirconium sulfate-containing reaction liquid to an alkali solution, the method is not particularly limited and either method may be used.

After the neutralization, the slurry is filtered to afford a zirconium-containing hydroxide. The zirconium-containing hydroxide is preferably washed with pure water or the like to remove impurities, as necessary. After washing with water, drying or the like may be performed, as necessary.

<Step 6>

In step 6, the zirconium-containing hydroxide obtained in step 5 is subjected to heat treatment (firing) to oxidize the zirconium-containing hydroxide, thereby affording a stabilized zirconia.

The heat treatment temperature (firing temperature) and the heat treatment time (firing time) of the zirconium-containing hydroxide are not particularly limited, and the heat treatment is usually performed at about 600 to 1050°C for 1 hour to 10 hours. The firing temperature is more preferably 650°C or higher and 1000°C or lower, and still more preferably 700°C or higher and 980°C or lower. The firing temperature is more preferably 2 hours to 6 hours, and still more preferably 2 hours to 4 hours. By setting the heat treatment temperature to 600°C or higher and 1200°C or lower, the specific surface area of the resulting stabilized zirconia can be set to a suitable range (for example, 5 $m^2/g$ or more and 60 $m^2/g$ or less). The heat treatment atmosphere is not particularly limited, and may be usually in the air or an oxidizing atmosphere.

<Step 7>

In step 7, stabilized zirconia obtained in step 6 is mixed with silver oxide having an average particle diameter of 0.5 μm or less. When mixing is performed, the stabilized zirconia powder and silver oxide may be formed into a slurry in order to homogenize these. A silver-containing stabilized zirconia powder is thus obtained.

<Step 8>

After step 7, the resulting silver-containing stabilized zirconia powder may be pulverized to form a slurry, as necessary. In this case, a binder may be added in order to improve moldability. When a slurry is not intended to form (is not intended to pulverize), the binder and the silver-containing stabilized zirconia powder may be uniformly mixed with a kneading machine.

The binder is preferably an organic binder. The organic binder is likely to be removed from the molded body in a heating furnace in an oxidizing atmosphere, and a degreased body can be obtained, whereby finally, impurities are less likely to remain in the sintered body.

Examples of the organic binder include those that are soluble in alcohol, or those that are soluble in mixed liquids of two or more selected from the group consisting of alcohols, water, aliphatic ketones, and aromatic hydrocarbons. Examples of the organic binder include at least one selected from the group consisting of polyethylene glycol, glycol fatty acid ester, glycerol fatty acid ester, polyvinyl butyral, polyvinyl methyl ether, polyvinyl ethyl ether, and vinyl propionate. The organic binder may further contain one or more thermoplastic resins that are insoluble in alcohols, or the mixed liquids.

After the addition of the organic binder, a target silver-containing stabilized zirconia powder can be obtained by performing such treatment as drying or pulverization by applying a publicly-known method.

The particle diameter $D_{50}$ of the silver-containing stabilized zirconia powder can be controlled by the pulverization of step 8. For example, pulverization is performed according to the state of the silver-containing stabilized zirconia powder obtained in step 5, and the particle diameter $D_{50}$ of the silver-containing stabilized zirconia powder can thereby be controlled.

The silver-containing stabilized zirconia powder immediately after step 7 is a silver-containing stabilized zirconia powder containing stabilized zirconia and silver oxide, and generally does not contain silver. After step 7, heating is performed to decompose all or part of silver oxide into silver. For example, after step 7, all or part of silver oxide is decomposed by the heat generated during drying into silver.

As described above, according to the method for producing a silver-containing stabilized zirconia powder according to the present embodiment,

(1) If heating is not performed after step 7, a silver-containing stabilized zirconia powder, which contains stabilized zirconia; and silver oxide having an average particle diameter of 0.5 μm or less but does not contain silver, is obtained,

(2) If heating is performed to a degree at which part of silver oxide is decomposed after step 7, a silver-containing stabilized zirconia powder containing stabilized zirconia; and silver and silver oxide, which have an average particle diameter of 0.5 μm or less, is obtained,

(3) If heating is performed to a degree at which all of silver oxide is decomposed after step 7, a silver-containing stabilized zirconia powder, which contains stabilized zirconia; and silver having an average particle diameter of 0.5 μm or less but does not contain silver oxide, is obtained.

In a case where a sintering aid, a colorant, and the like are added, a silver-containing stabilized zirconia powder containing the sintering aid, the colorant, and the like can be obtained by adding these and performing mixing in step 7. As a more detailed method of the mixing, it is preferable to disperse the mixture in pure water or the like to form a slurry, followed by wet-mixing.

In a case where step 7 is performed, a sintering aid, a colorant, and the like may be added when step 7 is carried out.

The silver-containing stabilized zirconia powder according to the present embodiment has been described above.

[Method for producing zirconia sintered body]

Hereinafter, an example of a method for producing a zirconia sintered body will be described. However, the method for producing a zirconia sintered body of the present invention is not limited to the following examples.

The method for producing a zirconia sintered body according to the present embodiment comprises: step X of molding the silver-containing stabilized zirconia powder to obtain a molded body; and

step Y of sintering the molded body at 1250°C or higher and 1500°C or lower and for 1 hour or more and 5 hours or less after the step X.

In the method for producing a zirconia sintered body according to the present embodiment, first, a silver-containing stabilized zirconia powder is prepared. As the silver-containing stabilized zirconia powder, one described in the section of [silver-containing stabilized zirconia powder] can be used.

Next, the silver-containing stabilized zirconia powder is molded to afford a molded body (step X). For the molding, a commercially available molding machine and a cold isostatic pressing method (CIP) can be employed. The silver-containing stabilized zirconia powder may be temporarily molded by a molding machine and then main-molded by press molding. The press molding may usually be in a range of 0.1 t to 3 t/cm$^2$. The pressure is preferably 0.5 t to 2.5 t/cm$^2$, more preferably 0.8 t to 2.2 t/cm$^2$, and still more preferably 1 t to 2 t/cm$^2$.

Next, the green compact is sintered under conditions of 1250°C or more and 1500°C or less and 1 hour or more and 5 hours or less (step Y).

In the present embodiment, since the silver-containing stabilized zirconia powder is used, the sintering temperature can be set to a low temperature of 1250°C to 1500°C. The sintering temperature is more preferably 1300°C or more and 1450°C or less, particularly preferably 1350°C or more and 1400°C or less. The holding time during sintering is not particularly limited, but is, for example, preferably about 1 to 5 hours, more preferably 1 hour to 3 hours. The sintering atmosphere may be air or an oxidizing atmosphere. The sintering may be performed under ordinary pressure, and it is not particularly necessary to apply pressure.

In a case where silver oxide remains in the silver-containing stabilized zirconia powder, all of the silver oxide is thermally decomposed into silver by this sintering step (step Y). Depending on the sintering conditions, part (for example, a very small amount) of silver oxide may remain in some cases.

In this manner, it is possible to obtain a silver-containing zirconia sintered body, which contains silver particles, zirconia, and a stabilizer and in which the average particle diameter of the silver particles is 0.5 μm or less, the average crystal grain size of the stabilized zirconia is 0.35 μm or less, and the relative sintered density is 98% or more.

The method for producing the zirconia sintered body according to the present embodiment has been described above.

EXAMPLES

Hereinafter, the present invention will be described in detail with reference to Examples, but the present invention is not limited to the following Examples as long as the gist thereof is not deviated. The silver-containing stabilized zirconia powder and the silver-containing zirconia sintered body in each of Examples and Comparative Examples contain hafnium oxide as an unavoidable impurity in an amount of 1 to 3 mass% based on zirconium oxide (calculated by the following Formula (X)).

([Mass of hafnium oxide]/([mass of zirconium oxide] + [mass of hafnium oxide])) × 100 (%)          <Formula (X)>

The maximum value and the minimum value of the content of each component shown in the following Examples should be considered as a preferable minimum value and a preferable maximum value of the present invention regardless of the content of other components.

In addition, the maximum value and the minimum value of the measured values shown in the following Examples should be considered to be the preferred minimum value and maximum value of the present invention regardless of the content (composition) of each component.

[Production of silver-containing stabilized zirconia powder]

(Example 1)

First, 213 g of a 25% by mass aqueous sodium sulfate solution and 450 g of an aqueous zirconium oxychloride solution having a concentration of 16% by mass in terms of $ZrO_2$ (acid concentration: 1 N) were separately heated to 95°C (step 1).

Then, the heated aqueous solutions were brought into contact with each other over 2 minutes so that the mass ratio $SO_4^{2-}/ZrO_2$ in the mixed liquid was 0.50 (step 2).

Next, the obtained basic zirconium sulfate-containing reaction liquid was aged by maintaining the reaction liquid at 95°C for 4 hours to obtain basic zirconium sulfate (step 3).

Next, the aged solution was cooled to room temperature, and then an aqueous yttrium chloride solution having a concentration of 10% by mass in terms of $Y_2O_3$ was added so that the concentration of $Y_2O_3$ was 3.2 mol%, and the resultant was uniformly mixed (step 4).

Next, a 25% by mass aqueous sodium hydroxide solution was added to the obtained mixed solution to neutralize the mixed solution until the pH reached 13 or more so that a hydroxide precipitate was formed (step 5).

Specifically, the operation was performed using an apparatus as described with reference to Fig. 1.

The obtained hydroxide precipitate was filtered and washed thoroughly with water. The obtained hydroxide was subjected to heat treatment at 1000°C (calcination temperature) in the air for 4 hours to afford a stabilized zirconia (yttria-stabilized zirconia powder) that was not pulverized (step 6).

Silver oxide powder (manufactured by Mitsuwa Chemical Co., Ltd., product name: silver(I) oxide, average particle diameter: 20 μm) was added to the obtained stabilized zirconia that was not pulverized at 1% by mass with respect to stabilized zirconia. Furthermore, alumina powder having an average primary particle diameter of 0.1 μm was added at 0.5% by mass with respect to stabilized zirconia, and the mixture was pulverized and mixed for 40 hours by a wet ball mill using water as a dispersion medium (step 7). The pulverization was performed using φ 5-mm zirconia beads. The zirconia slurry obtained after pulverization was dried at 110°C to obtain the silver-containing stabilized zirconia powder described in Example 1.

(Examples 2 to 5)

Silver-containing stabilized zirconia powders according to Examples 2 to 5 were obtained in the same manner as in Example 1, except that the amount of silver oxide added was changed as shown in Table 1, alumina powder was not added, and the calcination temperature was changed to 850°C.

(Example 6)

A silver-containing stabilized zirconia powder according to Example 6 was obtained in the same manner as in Example 1 except that alumina powder was not added and the calcination temperature was changed to 1150°C.

(Example 7)

A silver-containing stabilized zirconia powder according to Example 7 was obtained in the same manner as in Example 1 except that alumina powder was not added and the calcination temperature was changed to 830°C.

(Example 8)

The same powder as the silver-containing stabilized zirconia powder of Example 3 was used as the silver-containing stabilized zirconia powder of Example 8.

(Examples 9 and 10)

Silver-containing stabilized zirconia powders according to Examples 9 and 10 were obtained in the same manner as in Example 1, except that the amount of $Y_2O_3$ added was changed as shown in Table 1 and the calcination temperature was changed to 850°C.

(Example 11)

A silver-containing stabilized zirconia powder according to Example 11 was obtained in the same manner as in Example 1 except that an aqueous erbium chloride solution having a concentration of 10% by mass in terms of $Er_2O_3$ was added so that the $Er_2O_3$ content was 3.2 mol% instead of adding an aqueous yttrium chloride solution, and the calcination temperature was changed to 850°C.

(Example 12)

A silver-containing stabilized zirconia powder according to Example 12 was obtained in the same manner as in Example 1 except that an aqueous cerium chloride solution having a concentration of 12% by mass in terms of $CeO_2$ was added so that the $CeO_2$ content was 12 mol% instead of adding an aqueous yttrium chloride solution, and the amount of the alumina powder added was changed to 0.25% by mass.

(Example 13)

A silver-containing stabilized zirconia powder according to Example 13 was obtained in the same manner as in Example 1 except that an aqueous yttrium chloride solution was added so that the $Y_2O_3$ content was 0.9 mol% and calcium carbonate ($CaCO_3$) was added so that the content thereof was 2.1 mol% in terms of CaO instead of adding an aqueous yttrium chloride solution having a concentration of 10% by mass in terms of $Y_2O_3$ so that the $Y_2O_3$ content was 3.2 mol%, alumina powder was not added, and the calcination temperature was changed to 950°C.

(Example 14)

A silver-containing stabilized zirconia powder according to Example 14 was obtained in the same manner as in Example 1 except that the amount of alumina powder added was changed to 20% by mass and the calcination temperature was changed to 850°C.

(Comparative Example 1)

First, 213 g of a 25% by mass aqueous sodium sulfate solution and 450 g of an aqueous zirconium oxychloride solution having a concentration of 16% by mass in terms of $ZrO_2$ (acid concentration: 1 N) were separately heated to 95°C (step 1).

Then, the heated aqueous solutions were brought into contact with each other over 2 minutes so that the mass ratio $SO_4^{2-}/ZrO_2$ in the mixed liquid was 0.50 (step 2).

Next, the obtained basic zirconium sulfate-containing reaction liquid was aged by maintaining the reaction liquid at 95°C for 4 hours to obtain basic zirconium sulfate (step 3).

Next, the aged solution was cooled to room temperature, and then an aqueous yttrium chloride solution having a concentration of 10% by mass in terms of $Y_2O_3$ was added so that the concentration of $Y_2O_3$ was 3.2 mol%, and the resultant was uniformly mixed (step 4).

Next, a 25% by mass aqueous sodium hydroxide solution was added to the obtained mixed solution to neutralize the mixed solution until the pH reached 13 or more so that a hydroxide precipitate was formed (step 5).

Specifically, the operation was performed using an apparatus as described with reference to Fig. 1.

The obtained hydroxide precipitate was filtered and washed thoroughly with water. The obtained hydroxide was subjected to heat treatment at 850°C (calcination temperature) in the air for 4 hours to afford a stabilized zirconia (yttria-stabilized zirconia powder) that was not pulverized (step 6).

Silver powder (manufactured by Mitsuwa Chemical Co., Ltd., product name: silver (particles), particle diameter: 2 to 4 μm) was added to the obtained stabilized zirconia that was not pulverized at 1% by mass with respect to stabilized zirconia, and the mixture was pulverized and mixed for 40 hours by a wet ball mill using water as a dispersion medium (step 7). The pulverization was performed using φ 5-mm zirconia beads. The zirconia slurry obtained after pulverization was dried at 110°C to obtain the silver-containing stabilized zirconia powder according to Example 1.

(Comparative Example 2)

Heat treatment was performed on 99% silver (powder) (up to 525 mesh) manufactured by Mitsuwa Chemical Co., Ltd. at 400°C for 2 hours. The particle diameter was thus coarsened. A silver-containing stabilized zirconia powder according to Comparative Example 2 was obtained in the same manner as in Example 3, except that the obtained silver powder was used.

[Composition measurement of silver-containing stabilized zirconia powder]

The composition (in terms of oxide) of the silver-containing stabilized zirconia powder of each of Examples and Comparative Examples was analyzed using ICP-AES ("ULTIMA-2" manufactured by HORIBA, Ltd.). The results are shown in Table 1.

[Measurement of particle diameter of silver particles and/or silver oxide particles in silver-containing stabilized zirconia powder]

A COMPO image of the silver-containing stabilized zirconia powder of each of Examples and Comparative Examples was acquired using a SEM "Phenom Pro-x" (manufactured by Phenom-World). The Heywood diameter of the silver oxide particles or silver particles was measured using image analysis software "Image-Pro" (developed by Media Cybernetics). Specifically, the outline of the silver particle was manually surrounded (traced) on the image analysis software, and the area of the surrounded silver particle was measured and then converted into the Heywood diameter. A total of 10 Heywood diameters were measured, and the average particle diameter was calculated. The results are shown in Table 1.

[Measurement of specific surface area of silver-containing stabilized zirconia powder]

The specific surface area of the silver-containing stabilized zirconia powder of each of Examples and Comparative Examples was measured by a BET method using a specific surface area meter ("Macsorb" manufactured by Mountec Co., Ltd.). The results are shown in Table 1.

[Measurement of particle diameter $D_{50}$ of silver-containing stabilized zirconia powder]

First, 0.15 g of the silver-containing stabilized zirconia powder of each of Examples and Comparative Examples and 40 ml of a 0.2% aqueous sodium hexametaphosphate solution were placed in a 50 ml beaker, the silver-containing stabilized zirconia powder was dispersed by an ultrasonic homogenizer "Sonifier S-450D" (Emerson Japan, Ltd.) for 5 minutes, and then the resultant was injected into a device (laser diffraction-type particle size distribution measuring device ("SALD-2300" manufactured by Shimadzu Corporation)) for measurement. The results are shown in Table 1.

[Measurement of pore volume of silver-containing stabilized zirconia powder]

The pore distribution of the silver-containing stabilized zirconia powder of each of Examples and Comparative Examples was obtained by a mercury intrusion method using a pore distribution measuring device ("Autopore IV9500" manufactured by Micromeritics). The measurement conditions were as follows.

<Measurement conditions>

Measuring device: Pore distribution measuring device (Autopore IV9500 manufactured by Micromeritics)
Measuring range: 0.0036 to 10.3 $\mu$m
Number of measurement points: 120 points
Mercury contact angle: 140 degrees
Mercury surface tension: 480 dyne/cm

Using the acquired pore distribution, the peak top diameter, pore volume, and pore distribution width in a range of 10 nm or more and 0.5 $\mu$m or less were determined. The results are shown in Table 1.
Here, the pore distribution width refers to the width of a peak at which the log differential pore volume is 0.1 ml/g or more.

[Production of zirconia sintered body]

First, a green compact was obtained from the zirconia powder of each of Examples and Comparative Examples by cold isostatic pressing (CIP). The compacting pressure was set to 2 t/cm$^2$.

At this time, the relative compacted density of the green compact was determined as follows. The results are shown in Table 2.

Next, the green compact was sintered at the "sintering temperature" shown in Table 2 for 2 hours to obtain a silver-containing zirconia sintered body.

The silver-containing zirconia sintered body of Comparative Example 3 is a silver-containing zirconia sintered body obtained by sintering the silver-containing stabilized zirconia powder of Comparative Example 2 at a sintering temperature of 1550°C. In a case where silver particles are large, such as in the silver-containing stabilized zirconia powder of Comparative Example 2, sintering at 1550°C is required for densification, but it can be seen that the silver-containing zirconia sintered body of Comparative Example 3 obtained by sintering the silver-containing stabilized zirconia powder of Comparative Example 2 at a sintering temperature of 1550°C is poor in resistance to hydrothermal degradation since the sintering temperature was increased.

<Relative sintered density>

The relative sintered density refers to a relative sintered density represented by the following Formula (1).

$$\text{Relative sintered density (\%) = (sintered density/theoretical sintered density)} \times 100 \qquad (1)$$

Here, the theoretical sintered density (denoted by $\rho_0$) is a value calculated by the following Formula (2-1).

$$\rho_0 = (100 + Y + Z)/[(100/pz) + (Y/3.987) + (Z/10.49)] \qquad (2\text{-}1)$$

where Y is the alumina concentration (% by weight) and Z is the silver concentration (% by weight). In a case where silver oxide is used as a raw material, the silver concentration (% by weight) is calculated by Formula (2-2).

$$Z = [107.868 \times \text{(silver oxide concentration (\% by weight))}]/(231.736 \times 2) \qquad (2\text{-}2)$$

in which $\rho z$ is a value calculated by the following Formula (2-3).

$$\rho z = [124.25(100 - X) + [\text{molecular weight of stabilizer}] \times X]/150[150.5(100 + X)A^2C \qquad (2\text{-}3)$$

Here, as the molecular weight of the stabilizer, 225.81 is used when the stabilizer is $Y_2O_3$, 382.52 is used when the stabilizer is $Er_2O_3$, and 394.11 is used when the stabilizer is $Yb_2O_3$.

X is the stabilizer concentration (mol%). A and C are values calculated by the following Formulas (2-4) and (2-5), respectively.

$$A = 0.5080 + 0.06980X/(100 + X) \quad \dots \quad (2\text{-}4)$$

$$C = 0.5195 - 0.06180X/(100 + X) \quad \dots \quad (2\text{-}5)$$

In Formula (1), the theoretical sintered density varies depending on the composition of the powder.

For example, the theoretical sintered density of yttria-containing zirconia is 6.117 g/cm$^3$ when the yttria content is 2 mol%, 6.098 g/cm$^3$ when the yttria content is 3 mol%, and 6.051 g/cm$^3$ when the yttria content is 5.5 mol% (in the case of $Al_2O_3$ = 0% by weight).

When the stabilizer is $Sc_2O_3$, $\rho z$ is a value calculated by the following Formula (3).

$$\rho z = -0.0402 \text{ (molar concentration of } Sc_2O_3) + 6.1294 \qquad (3)$$

When the stabilizer is CaO, $\rho z$ is a value calculated by the following Formula (3-1).

$$\rho z = -0.0400(\text{molar concentration of CaO}) + 6.1700 \ldots$$

(3-1)

The theoretical sintered density (denoted by $\rho 1$) in the case of containing a colorant is

$$\rho 1 = 100/[(Z/V) + (100 - Z)/\rho 0] \ldots (2\text{-}5)$$

Z is the concentration (% by weight) of the colorant, and V is the theoretical density (g/cm$^3$) of the colorant.
The theoretical density of the colorant is 5.24 g/cm$^3$ for $Fe_2O_3$, 5.61 g/cm$^3$ for ZnO, 5.03 g/cm$^3$ for $MnO_2$, 6.10 g/cm$^3$ for CoO, 5.22 g/cm$^3$ for $Cr_2O_3$, 4.23 g/cm$^3$ for $TiO_2$, 7.80 g/cm$^3$ for $Tb_4O_7$, 6.31 g/cm$^3$ for CuO, and 3.36 g/cm$^3$ for $V_2O_5$.
The sintered density was measured by Archimedes method.

<Relative compacted density>

Relative compacted density (%) = (compacted density/theoretical sintered density) $\times$ 100　　　　(4)

Here, the theoretical sintered density (denoted by $\rho_0$) is a value calculated by Formula (2-1).

[Particle diameter of silver particles in silver-containing zirconia sintered body]

The surface of the sintered body of each of Examples and Comparative Examples was ground and polished to expose the portion containing silver, and a COMPO image was acquired using a SEM "Phenom Pro-x" (manufactured by Phenom-World). The Heywood diameter of silver particles was measured using image analysis software "Image-Pro" (developed by Media Cybernetics). Specifically, the outline of the silver particle was manually surrounded (traced) on the image analysis software, and the area of the surrounded silver particle was measured and then converted into the Heywood diameter. A total of 100 Heywood diameters were measured, and the average particle diameter of silver particles was calculated. The results are shown in Table 2.

[Average crystal grain size of stabilized zirconia in silver-containing zirconia sintered body]

The crystal grain size of stabilized zirconia in the zirconia sintered body of each of Examples and Comparative Examples was determined as follows. The results are shown in Table 2.
The SEM observation image of the obtained sintered body sample was acquired by scanning electron microscopy. The sample for SEM observation was prepared in accordance with JIS R 1633. The number of crystal grains in stabilized zirconia per visual field of the SEM observation image was set to 150 or more. A rectangle of any size was drawn in the SEM observation image, and the number of grains present on the sides and diagonal lines of the rectangle was counted. In Examples and Comparative Examples, the magnification was set to 30,000x, and a rectangle of 3.497 $\mu$m $\times$ 2.375 $\mu$m was drawn. The length of each of the sides of the rectangle was set to be equal to or more than 80% of the visual field. The average crystal grain size was calculated from the numbers of grains and the lengths of four sides and diagonal lines of the rectangle.
Specifically, the average crystal grain size was calculated by the following formula.

(Average crystal grain size) = {[X/(x1 + x2)] + [Y/(y1 + y2)] + [D/(d1 + d2)]} $\times$ 2/3

X, x1, x2, Y, y1, y2, D, d1, and d2 in the formula denote the following.

X ($\mu$m): Length of long side of rectangle
Y ($\mu$m): Length of short side of rectangle
D ($\mu$m): Length of diagonal line of rectangle
x1 (number of grains): Number of grains on one of long sides
x2 (number of grains): Number of gains on the other long side
y1 (number of grains): Number of grains on one of short sides

23

y2 (number of grains): Number of grains on the other short side
d1 (number of grains): Number of grains on one of diagonal lines
d2 (number of grains: Number of grains on the other diagonal line

The same operation was performed for three visual fields at each level, and the average of average crystal grain sizes of the three visual fields was defined as a final average crystal grain size. The results are shown in Table 2.

Prior to the measurement, the sintered body sample was pretreated by performing mirror polishing and then thermal etching. The mirror polishing was performed by grinding the surface of the sintered body with a surface grinder and then polishing this with a mirror polishing device while changing the average particle diameter of diamond abrasive grains used in the order of 9 $\mu$m, 6 $\mu$m, and 3 $\mu$m.

[Monoclinic fraction of silver-containing zirconia sintered body after hydrothermal treatment at 134°C and 0.3 MPa for 15 hours]

First, the silver-containing zirconia sintered body of each of Examples and Comparative Examples obtained above was subjected to hydrothermal treatment at 134°C and an absolute pressure of 0.3 MPa (in an underwater environment) for 15 hours. Thereafter, the fraction of the monoclinic phase contained in the crystal phase of the silver-containing zirconia sintered body after hydrothermal treatment was determined.

The silver-containing zirconia sintered body of each of Examples and Comparative Examples after hydrothermal treatment was mirror-polished, and the fraction of the monoclinic phase contained in the crystal phase was determined. The mirror polishing was performed by grinding the surface of the sintered body with a surface grinder and then polishing this with a mirror polishing device while changing the average particle diameter of diamond abrasive grains used in the order of 9 $\mu$m, 6 $\mu$m, and 3 $\mu$m. The results are shown in Table 2.

Specifically, the monoclinic fraction of the silver-containing zirconia sintered body of each of Examples and Comparative Examples was determined in the manner described below in [Identification of crystal phase].

[Identification of crystal phase]

The X-ray diffraction spectrum of the silver-containing zirconia sintered body was acquired using an X-ray diffractometer ("RINT2500" manufactured by Rigaku Corporation). The measurement conditions were as follows.

<Measurement conditions>

Measuring device: X-ray diffractometer (RINT2500, manufactured by Rigaku Corporation)
Radiation source: CuK$\alpha$ radiation source
Sampling interval: 0.02°
Scanning speed: 2$\theta$ = 1.0°/min
Divergence slit (DS): 1°
Divergence vertical limit slit: 5 mm
Scatter slit (SS): 1°
Receiving slit (RS): 0.3 mm
Monochrome receiving slit: 0.8 mm
Tube voltage: 50 kV
Tube current: 300 mA
Scanning speed: 2$\theta$ = 26 to 36°: 4°/min
2$\theta$ = 72 to 76°: 1°/min

Then, the crystal phases were identified from the X-ray diffraction spectrum. The respective phase fractions of crystal phases contained in the silver-containing zirconia sintered body were calculated by the following formulae.

Monoclinic fraction (%) = (Im(111) + Im(11- 1))/(Im(111) + Im(11-1) + It(101) + Ic(111)) $\times$ 100

Tetragonal fraction (%) = (100% - monoclinic phase (%)) $\times$ ((It(004) + It(220)/(It(004) + It(220) + Ic(004)) $\times$ 100

Cubic fraction (%) = (100% - monoclinic phase (%)) $\times$ ((Ic(004)/(It(004) + It(220) + Ic(004)) $\times$ 100

Here, Im(111) is the diffraction intensity of (111) in the monoclinic phase, and Im(11-1) is the diffraction intensity of (11-1) in the monoclinic phase.

It(101) is the diffraction intensity of (101) in the tetragonal phase, It(220) is the diffraction intensity of (220) in the tetragonal phase, and It(004) is the diffraction intensity of (004) in the tetragonal phase.

Ic(004) is the diffraction intensity of (004) in the cubic phase and Ic(111) is the diffraction intensity of (111) in the cubic phase.

The monoclinic phase of zirconia was discriminated from the tetragonal phase and the cubic phase in the vicinity of $2\theta = 26$ to $36°$ in the XRD spectrum. The tetragonal phase was discriminated from the cubic phase in the vicinity of $2\theta = 72$ to $76°$ in the XRD spectrum. The cubic phase may be distorted depending on the amount of the stabilizer added and the type of production method used, which may cause a peak position to shift. However, in the present Examples, a peak between (004) and (220) in the tetragonal phase was calculated as the peak of the cubic phase.

[Toughness]

The toughness of the silver-containing zirconia sintered body of each of Examples and Comparative Examples obtained above were determined as follows.

The measurement of toughness based on the IF method was performed by a method in accordance with JIS R 1607 (Testing methods for fracture toughness of fine ceramics at room temperature) using a load of 30 kgf (294.2 N). Seven quadrangular indentations formed by a Vickers hardness tester were selected to determine toughness values, and the average of five toughness values excluding the minimum and maximum values was defined as a toughness value. Indentations from which cracks were not extended were not used for measurement, and rectangular indentations having four tips from which cracks were extended were used.

The toughness values were calculated by the following formula.

$$Kc = 0.018 \times Hv \times a^{0.5} \times [(c - a)/a]^{-0.5} \times (Hv/E)^{-0.4}$$

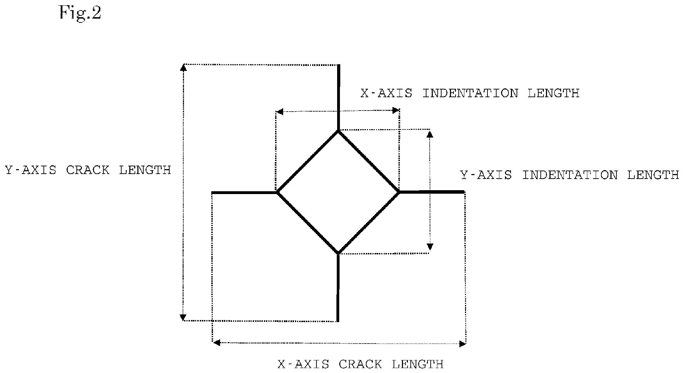

Kc, Hv, a, c, and E mean the following. The indentation lengths along the X and Y axes and the crack lengths along the X and Y axes for determining a and c are as shown in Fig. 2.

Kc: Toughness value [MPa·m$^{0.5}$]
Hv: Vickers hardness [GPa]
a: Half of average of indentation lengths along X and Y axes [μm]
c: Half of average of crack lengths along X and Y axes [μm]
E: Young's modulus [GPa]

The Vickers hardness was determined in accordance with JIS R 1610 (Test methods for hardness of fine ceramics). The Vickers hardness was calculated by the following formula.

$$Hv = 0.001854 \times [F/d^2 Sv]$$

F and d mean the following. The X-axis indentation length and the Y-axis indentation length for determining d are as shown in Fig. 2.

Hv: Vickers hardness [GPa]
F: Test force [N]
d: Average [mm] of X-axis indentation length and Y-axis indentation length
As the Young's modulus, 210 GPa, which is known as a Young's modulus of common yttria-stabilized zirconia, was used.

[Three-point bending strength]

The three-point bending strength of the silver-containing zirconia sintered body of each of Examples and Comparative Examples obtained above was measured in accordance with three-point bending strength specified in JIS R 1601. The results are shown in Table 2.

[Table 1]

| Distinction | Raw material of silver | | Stabilizer | | | | $Al_2O_3$ | Powder properties | | | | | |
|---|---|---|---|---|---|---|---|---|---|---|---|---|---|
| | Kind | Added amount [wt%] | Kind (1) | Added amount (1) [mol%] | Kind (2) | Added amount (2) [mol%] | Added amount [wt%] | Silver particle diameter [$\mu$m] | S.A. [m²/g] | $D_{50}$ [$\mu$m] | Peak top diameter [nm] | Pore distribution width [nm] | Pore volume [mL/g] |
| Example 1 | $Ag_2O$ | 1 | $Y_2O_3$ | 3.2 | - | - | 0.5 | 0.34 | 17.7 | 0.35 | 82 | 53 | 0.29 |
| Example 2 | $Ag_2O$ | 0.025 | $Y_2O_3$ | 3.2 | - | - | 0 | 0.17 | 31.2 | 0.55 | 43 | 54 | 0.30 |
| Example 3 | $Ag_2O$ | 4 | $Y_2O_3$ | 3.2 | - | - | 0 | 0.38 | 35.7 | 1.20 | 74 | 106 | 0.40 |
| Example 4 | $Ag_2O$ | 16 | $Y_2O_3$ | 3.2 | - | - | 0 | 0.33 | 30.9 | 0.46 | 61 | 113 | 0.36 |
| Example 5 | $Ag_2O$ | 20 | $Y_2O_3$ | 3.2 | - | - | 0 | 0.41 | 32.1 | 0.54 | 82 | 100 | 0.33 |
| Example 6 | $Ag_2O$ | 1 | $Y_2O_3$ | 3.2 | - | - | 0 | 0.21 | 9.1 | 0.60 | 186 | 185 | 0.20 |
| Example 7 | $Ag_2O$ | 1 | $Y_2O_3$ | 3.2 | - | - | 0 | 0.25 | 36.8 | 0.54 | 41 | 45 | 0.35 |
| Example 8 | $Ag_2O$ | 4 | $Y_2O_3$ | 3.2 | - | - | 0 | 0.38 | 35.7 | 1.20 | 74 | 106 | 0.40 |
| Example 9 | $Ag_2O$ | 1 | $Y_2O_3$ | 1.65 | | | 0.5 | 0.19 | 32.0 | 0.63 | 44 | 65 | 0.39 |
| Example 10 | $Ag_2O$ | 1 | $Y_2O_3$ | 5.6 | - | - | 0.5 | 0.25 | 32.7 | 0.55 | 65 | 57 | 0.29 |
| Example 11 | $Ag_2O$ | 1 | $Er_2O_3$ | 3.2 | - | - | 0.5 | 0.22 | 30.5 | 0.55 | 74 | 69 | 0.39 |
| Example 12 | $Ag_2O$ | 1 | $CeO_2$ | 12 | - | - | 0.25 | 0.39 | 14.8 | 0.34 | 83 | 50 | 0.29 |
| Example 13 | $Ag_2O$ | 1 | CaO | 2.1 | $Y_2O_3$ | 0.9 | 0 | 0.28 | 23.9 | 0.59 | 48 | 46 | 0.35 |
| Example 14 | $Ag_2O$ | 1 | $Y_2O_3$ | 3.2 | - | - | 20 | 0.15 | 26.9 | 0.39 | 69 | 73 | 0.37 |
| Comparative Example 1 | Ag | 4 | $Y_2O_3$ | 3.2 | - | - | 0 | 1342 | 34.0 | - | - | - | - |
| Comparative Example 2 | Ag | 4 | $Y_2O_3$ | 3.2 | - | - | 0 | 54 | 35.1 | 0.39 | 70 | 115 | 0.36 |

EP 4 563 548 A1

[Table 2]

| Distinction | Sintered body properties | | | | | | | |
|---|---|---|---|---|---|---|---|---|
| | Sintering temperature [°C] | Relative density [%] | Particle diameter of silver particle [μm] | Crystal grain size [μm] | m-Phase fraction before hydrothermal degradation [%] | m-Phase fraction after hydrothermal degradation [%] | Fracture toughness value [MPa$^{0.5}$] | Bending strength [MPa] |
| Example 1 | 1400 | 99.7 | 0.28 | 0.21 | Undetected | 5.5 | 5.3 | 895 |
| Example 2 | 1300 | 99.2 | 0.15 | 0.11 | Undetected | 0.3 | 5.1 | 990 |
| Example 3 | 1400 | 98.9 | 0.46 | 0.19 | Undetected | 8.5 | 5.5 | 987 |
| Example 4 | 1400 | 98.2 | 0.26 | 0.26 | Undetected | 7.9 | 6.0 | 913 |
| Example 5 | 1450 | 98.2 | 0.33 | 0.28 | Undetected | 11.8 | 5.6 | 821 |
| Example 6 | 1500 | 99.6 | 0.31 | 0.31 | Undetected | 77.1 | 5.2 | 976 |
| Example 7 | 1400 | 99.5 | 0.32 | 0.17 | Undetected | 3.8 | 5.1 | 1010 |
| Example 8 | 1500 | 99.5 | 0.25 | 0.34 | Undetected | 79.5 | 5.1 | 1073 |
| Example 9 | 1300 | 99.8 | 0.18 | 0.19 | 0.6 | 14.6 | 18.3 | 1121 |
| Example 10 | 1350 | 99.6 | 0.18 | 0.19 | Undetected | 0.2 | 5.1 | 605 |
| Example 11 | 1350 | 99.4 | 0.30 | 0.22 | Undetected | 1.0 | 5.5 | 1015 |
| Example 12 | 1350 | 99.6 | 0.28 | 0.34 | Undetected | 0.4 | 20.1 | 609 |
| Example 13 | 1350 | 99.4 | 0.26 | 0.11 | 0.4 | 7.2 | 15.9 | 1050 |
| Example 14 | 1500 | 99.7 | 0.24 | 0.32 | Undetected | 3.0 | 5.3 | 1005 |
| Comparative Example 1 | Cannot be evaluated as it cannot be compacted. | | | | | | | |
| Comparative Example 2 | 1500 | 97.8 | 11.77 | 0.25 | Undetected | 33.7 | Unmeasurable | 513 |
| Comparative Example 3 | 1550 | 99.2 | 13.53 | 0.53 | Undetected | 92.5 | Unmeasurable | 478 |

**Claims**

1. A silver-containing zirconia sintered body comprising:

   silver particles; and
   stabilized zirconia containing zirconia and a stabilizer, wherein
   an average particle diameter of the silver particles is 0.5 $\mu$m or less,
   an average crystal grain size of the stabilized zirconia is 0.35 $\mu$m or less, and
   a relative sintered density is 98% or more.

2. The silver-containing zirconia sintered body according to claim 1, wherein an average crystal grain size of the stabilized zirconia is 0.25 $\mu$m or less.

3. The silver-containing zirconia sintered body according to claim 1 or 2, wherein silver particles are contained in a range of 0.025% by mass or more and 20% by mass or less in terms of $Ag_2O$ when a sum of zirconia and a stabilizer is taken as 100% by mass.

4. The silver-containing zirconia sintered body according to claim 1 or 2, which has a three-point bending strength of 600 MPa or more.

5. The silver-containing zirconia sintered body according to claim 1 or 2, which has a toughness value of 5 MPa·m$^{0.5}$ or more as measured by an IF method.

6. The silver-containing zirconia sintered body according to claim 1 or 2, wherein a monoclinic fraction after hydrothermal treatment at 134°C and 0.3 MPa for 15 hours is 10% or less.

7. The silver-containing zirconia sintered body according to claim 1 or 2, wherein the stabilizer is one or more selected from the group consisting of $Y_2O_3$, $CeO_2$, $Er_2O_3$, and CaO.

8. The silver-containing zirconia sintered body according to claim 1 or 2, wherein a content of a stabilizer is 15 mol% or less when zirconia and the stabilizer are taken as the whole.

9. A silver-containing stabilized zirconia powder comprising:

   stabilized zirconia containing zirconia and a stabilizer; and
   silver and/or silver oxide having an average particle diameter of 0.5 $\mu$m or less.

10. The silver-containing stabilized zirconia powder according to claim 9, wherein a peak top diameter in pore volume distribution is 30 nm or more and 100 nm or less, a pore volume is 0.25 ml/g or more and 0.46 ml/g or less, and a pore distribution width is 40 nm or more and 120 nm or less in a range of 10 nm or more and 0.5 $\mu$m or less in pore distribution based on a mercury intrusion method.

11. A method for producing a silver-containing stabilized zirconia powder, the method comprising a step of mixing stabilized zirconia containing zirconia and a stabilizer with silver oxide having an average particle diameter of 0.5 $\mu$m or less.

12. A method for producing a silver-containing zirconia sintered body, the method comprising:

    a step X of compacting the silver-containing stabilized zirconia powder according to claim 9 or 10 to obtain a green compact; and
    a step Y of sintering the green compact under conditions of 1250°C or more and 1500°C or less and 1 hour or more and 5 hours or less after the step X.

Fig.1

ZIRCONIUM SALT SOLUTION   10   12   L3   20   32   30   SULFATING AGENT SOLUTION

L1   L2

40   42

Fig.2

X-AXIS INDENTATION LENGTH

Y-AXIS CRACK LENGTH

Y-AXIS INDENTATION LENGTH

X-AXIS CRACK LENGTH

## INTERNATIONAL SEARCH REPORT

| International application No. |
| --- |
| **PCT/JP2024/011255** |

**A. CLASSIFICATION OF SUBJECT MATTER**

*C04B 35/488*(2006.01)i; *A61C 8/00*(2006.01)i; *A61K 6/818*(2020.01)i; *C01G 25/02*(2006.01)i
FI: C04B35/488; C01G25/02; A61C8/00 Z; A61K6/818

According to International Patent Classification (IPC) or to both national classification and IPC

**B. FIELDS SEARCHED**

Minimum documentation searched (classification system followed by classification symbols)

C04B35/488; A61C8/00; A61K6/818; C01G25/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | CN 105367056 A (DONGGUAN CSG CERAMICS TECHNOLOGY CO., LTD.) 02 March 2016 (2016-03-02) | 1-9, 11-12 |
| | paragraphs [0025]-[0027], [0043], [0051], [0053], [0068], [0073], [0076] | |
| A | entire text, all drawings | 10 |
| X | US 2016/0015483 A1 (OSSEODYNE SURGICAL SOLUTIONS, LLC.) 21 January 2016 (2016-01-21) | 1-8 |
| | paragraphs [0027], [0253], [0261] | |
| A | entire text, all drawings | 9-12 |
| A | JP 11-228320 A (HOKKO CHEMICAL INDUSTRY CO., LTD.) 24 August 1999 (1999-08-24) | 1-12 |
| | entire text, all drawings | |
| A | JP 2007-063530 A (SAGA PREFECTURE) 15 March 2007 (2007-03-15) | 1-12 |
| | entire text, all drawings | |

☑ Further documents are listed in the continuation of Box C. ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **24 May 2024** | **04 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/011255**

| C. | DOCUMENTS CONSIDERED TO BE RELEVANT | | |
|---|---|---|
| Category* | Citation of document, with indication, where appropriate, of the relevant passages | Relevant to claim No. |
| A | WO 2020/049299 A1 (CAMBRIDGE NANOCERAMIX LTD.) 12 March 2020 (2020-03-12) entire text, all drawings | 1-12 |

Form PCT/ISA/210 (second sheet) (July 2022)

**INTERNATIONAL SEARCH REPORT**
Information on patent family members

International application No.

**PCT/JP2024/011255**

| Patent document cited in search report | | | Publication date (day/month/year) | Patent family member(s) | Publication date (day/month/year) |
|---|---|---|---|---|---|
| CN | 105367056 | A | 02 March 2016 | (Family: none) | |
| US | 2016/0015483 | A1 | 21 January 2016 | WO 2015/168332 A2 paragraphs [0026], [0215], [0223] | |
| JP | 11-228320 | A | 24 August 1999 | (Family: none) | |
| JP | 2007-063530 | A | 15 March 2007 | (Family: none) | |
| WO | 2020/049299 | A1 | 12 March 2020 | US 2021/0338889 A1 entire text, all drawings | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KAIJIN XU**. Microorganism adhesion inhibited by silver doped Yttria-stabilized zirconia ceramics. *Ceram. Int.*, 2011, vol. 37, 2109-2115

- **YAMADA, R.** Ag nanoparticle-coated zirconia for antibacterial prosthesis. *Mater. Sci. Eng. C*, 2017, vol. 78, 1054-1060